# DEMANDE DE BREVET EUROPEEN

(11) **EP 2 644 190 A1**
(43) Date de publication de la demande: **02.10.2013**
(21) Numéro de dépôt: 13161881.1
(22) Date de dépôt: 29.03.2013
(51) Int. Cl.: A61K 9/107, A61K 31/00

(54) **Formulation pour l'hormonothérapie**

(30) Priorité: 30.03.2012 FR 1252919
(71) Demandeur: Commissariat à l'Énergie Atomique et aux Énergies Alternatives, 75015 Paris (FR)
(72) Inventeur: Merian, Juliette, 19100 Brive (FR); Boisgard, Raphaël, 91620 Nozay (FR); Tavitian, Bertrand, 75015 Paris (FR); Texier-Nogues, Isabelle, 38000 Grenoble (FR)
(74) Mandataire: Domenego, Bertrand

(57) **Abrégé**

La présente invention concerne une formulation sous forme de nanoémulsion, comprenant une phase aqueuse continue et au moins une phase dispersée, et comprenant :
- un lipide amphiphile,
- un lipide solubilisant,
- une hormone, un agoniste ou un antagoniste d'hormone ou un mélange de ceux-ci, et
- un co-tensioactif comprenant au moins une chaîne composée de motifs d'oxyde d'alkylène
pour son utilisation pour un traitement hormonal choisi parmi la contraception, l'aide à la procréation, le traitement hormonal post-ménopause, le traitement des ménorragies, le traitement de l'acné, et le traitement des dysrégulations surrénaliennes.

## Description

La présente invention concerne l'utilisation d'une formulation de type nanoémulsion, pour l'hormonothérapie.

Des systèmes de délivrance pour l'hormonothérapie sont décrits dans la littérature, avec des voies d'administration (orale, transdermique, intra-utérine ou intramusculaire) et des formes d'administration variées (comprimé, gel, émulsions, patch, implants, spray, anneau vaginal...) (Yoo et al., Journal of Controlled Release, 112, 2006, 1-14).

Quelques exemples de formulations sous forme de suspension ont été décrits. Par exemple, Salem (International Journal of Nanomedecine, 2010, 5, 943-954) rapporte l'utilisation d'un gel comprenant une nanosuspension dont les nanoparticules comprennent de la progestérone, du Pluronic F127 et de l'acide stéarique, administrable par voie intramusculaire et permettant de limiter la fréquence d'administration. Mittal et al. (journal of Pharmaceutical sciences, 98(10), 2009, 3730-3734) décrit des nanoparticules polymériques (à base de poly (lactide-co-glycolide)) comprenant de l'oestradiol pour l'administration orale permettant d'améliorer la biodisponibilité de l'oestradiol.

La voie orale reste la voie d'administration la plus utilisée pour le traitement hormonal. Or, comme la biodisponibilité des hormones est faible (principalement à cause d'un premier passage hépatique très important et de la métabolisation de l'hormone dans l'intestin), les doses d'hormones administrées sont élevées, ce qui entraîne des effets secondaires importants. Les patchs ont été développés comme voie d'administration alternative, mais la constance de la délivrance et les problèmes de pénétration de l'hormone restent des challenges.

Par ailleurs, la demande de brevet WO 2010/018223 décrit l'utilisation d'une formulation sous forme de nanoémulsion, comprenant une phase aqueuse continue et au moins une phase dispersée comprenant un lipide amphiphile, un lipide solubilisant, un agent thérapeutique et un co-tensioactif comprenant au moins une chaîne composée de motifs d'oxyde d'alkylène, pour la délivrance d'un agent thérapeutique. Cette demande ne suggère aucunement que cette formulation cible les organes stéroïdiens, et son utilisation pour le traitement hormonal n'est pas décrite.

Le développement de nouvelles méthodes de traitement hormonal est nécessaire, permettant de minimiser les effets secondaires et présentant une bonne efficacité, une bonne tolérance par les patients et une bonne biodisponibilité des principes actifs.

A cet effet, la présente invention fournit une formulation comprenant une hormone, un agoniste ou un antagoniste d'hormone, et ciblant préférentiellement les organes stéroïdiens, et qui est par conséquent utile pour l'hormonothérapie.

### [Définitions]

Au sens de la présente demande, les expressions « hormonothérapie » et « traitement hormonal » ont la même signification et signifient l'administration d'une hormone naturelle ou artificielle, que cette hormone soit inhibitrice d'une autre hormone (administration d'un antagoniste d'hormone) ou compensatrice (administration d'un agoniste d'hormone pour compenser un défaut de sécrétion pour une hormonothérapie de substitution).

Dans le cadre de cet exposé, on entend par le terme « nanoémulsion » une composition présentant au moins deux phases, généralement une phase huileuse et une phase aqueuse, dans laquelle la taille moyenne de la phase dispersée est inférieure à 1 micron, de préférence de 10 à 500 nm et en particulier de 20 à 100 nm, et tout préférentiellement de 20 à 70 nm (voir article C. Solans, P. Izquierdo, J. Nolla, N. Azemar and M. J. Garcia-Celma, Curr Opin Colloid In, 2005, 10, 102-110).

Au sens de la présente demande, l'expression « phase dispersée » signifie les gouttelettes comprenant l'éventuelle huile/le lipide solubilisant/ le lipide amphiphile/ le co-tensioactif/ l'hormone, l'agoniste ou l'antagoniste d'hormone ou le mélange de ceux-ci. La phase dispersée est généralement exempte de phase aqueuse.

Le terme « gouttelette » englobe à la fois les gouttelettes d'huile liquide proprement dites ainsi que les particules solides issues d'émulsions de type huile-dans-eau dans lesquelles la phase huileuse est solide. Dans ce dernier cas, on parle souvent aussi d'émulsion solide.

Le terme « lipide » désigne dans le cadre de cet exposé l'ensemble des corps gras ou des substances contenant des acides gras présents dans les graisses d'origine animales et dans les huiles végétales. Ce sont des molécules hydrophobes ou amphiphiles principalement constituées de carbone, d'hydrogène et d'oxygène et ayant une densité inférieure à celle de l'eau. Les lipides peuvent être à l'état solide à température ambiante (25°C), comme dans les cires, ou liquide, comme dans les huiles.

Le terme « phospholipide » vise des lipides possédant un groupe phosphate, notamment les phosphoglycérides. Le plus souvent, les phospholipides comportent une extrémité hydrophile formée par le groupe phosphate éventuellement substitué et deux extrémités hydrophobes formées par des chaînes d'acides gras. Parmi les phospholipides, on citera en particulier la phosphatidylcholine, la phosphatidyl éthanolamine, la phophatidyl inositol, la phosphatidyl sérine et la sphingomyéline.

Le terme « lécithine » désigne la phosphatidylcholine, c'est-à-dire un lipide formé à partir d'une choline, d'un phosphate, d'un glycérol et de deux acides gras. Il couvre de manière plus large les phospholipides extraits du vivant, d'origine végétale ou animale, dans la mesure où ils sont majoritairement constitués de phosphatidylcholine. Ces lécithines constituent généralement des mélanges de lécithines portant différents acides gras.

On entend par le terme « acide gras » désigner des acides carboxyliques aliphatiques présentant une chaîne carbonée d'au moins 4 atomes de carbone. Les acides gras naturels possèdent une chaîne carbonée de 4 à 28 atomes de carbone (généralement un nombre pair). On parle d'acide gras à longue chaîne pour une longueur de 14 à 22 carbones et à très longue chaîne s'il y a plus de 22 carbones.

On entend par le terme « tensioactif » des composés à structure amphiphile qui leur confère une affinité particulière pour les interfaces de type huile/eau et eau/huile ce qui leur donne la capacité d'abaisser l'énergie libre de ces interfaces et de stabiliser des systèmes dispersés.

On entend par le terme « co-tensioactif » un tensioactif agissant en plus d'un tensioactif pour abaisser davantage l'énergie de l'interface.

On entend par le terme « chaîne hydrocarbonée » désigner une chaîne composée d'atomes de carbone et d'hydrogène, saturée ou insaturée (double ou triple liaison). Les chaînes hydrocarbonées préférées sont les alkyle ou alcényle.

On entend par le terme « alkylène » désigner un groupe divalent aliphatique hydrocarboné, saturé, linéaire ou ramifié, de préférence linéaire.

Par « ester activé », on entend un groupe de formule -CO-LG, et par « carbonate activé », on entend un groupe de formule -O-CO-LG où LG est un bon groupe partant notamment choisi parmi un chlore, un imidazolyle, un pentafluorophénolate, un pentachlorophénolate, un 2,4,5-trichlorophénolate, 2,4,6-trichlorophénolate, un groupe -O-succinimidyle, -O-benzotriazolyle, -O-(7-aza-benzotriazolyle) et -O-(4-nitrophényle).

### [Formulation]

Selon un premier objet, l'invention concerne une formulation sous forme de nanoémulsion, comprenant une phase aqueuse continue et au moins une phase dispersée, et comprenant :
- un lipide amphiphile,
- un lipide solubilisant,
- une hormone, un agoniste ou un antagoniste d'hormone ou un mélange de ceux-ci, et
- un co-tensioactif comprenant au moins une chaîne composée de motifs d'oxyde d'alkylène,
pour son utilisation pour le traitement hormonal, également appelé hormonothérapie.

L'émulsion est donc une émulsion de type huile dans l'eau. Elle peut être simple ou multiple, notamment en comportant dans la phase dispersée une seconde phase aqueuse. De préférence, elle est simple.

La contraception, l'aide à la procréation, le traitement hormonal post-ménopause (également appelé traitement hormonal substitutif (THS)), le traitement des ménorragies, le traitement de l'acné, et le traitement des dysrégulations surrénaliennes telles que la maladie d'Addison, la fatigue surrénalienne et l'infertilité sont les traitements hormonaux les plus courants.

L'invention concerne également l'utilisation de la formulation ci-dessus pour la contraception, le traitement hormonal post-ménopause ou l'aide à la procréation.

L'invention repose sur la découverte inattendue que la formulation décrite ci-dessus cible spécifiquement les organes stéroïdiens tels que les ovaires et les surrénales. Par conséquent, la formulation est particulièrement adaptée pour être utilisée pour l'hormonothérapie, où la délivrance d'hormone, d'agoniste ou d'antagoniste d'hormone au niveau de ces organes est souhaitée.

La formulation utilisée présente également d'autres avantages qui la rendent intéressante pour son utilisation thérapeutique.

Tout d'abord, le potentiel zêta de la phase dispersée est inférieur à 20 mV en valeur absolue, c'est-à-dire compris entre -20 et 20 mV. Un potentiel zéta élevé en valeur absolue des gouttelettes a pour effet l'accumulation dans certains organes, notamment dans le foie, la rate, les poumons, en plus des reins. La formulation utilisée est donc particulièrement adaptée à être administrée dans le corps car elle ne s'accumule pas dans ces organes.

Comme explicité ci-dessous, elle présente avantageusement une bonne biodisponibilité et peut permettre de limiter voire éviter les effets secondaires liés à l'administration d'hormone, agoniste ou antagoniste d'hormone généralement observés avec d'autres systèmes de délivrance.

De plus, la formulation utilisée dans l'invention est avantageusement stable : elle peut être stockée plus de trois mois sans qu'aucune dégradation ne soit observée.

### ● hormone et agoniste ou antagoniste d'hormone

Un traitement hormonal consiste à administrer une hormone, ou un agoniste ou antagoniste d'hormone, qui permet de moduler l'activité d'un récepteur d'une hormone, en l'activant (pour un agoniste) ou en l'inhibant (pour un antagoniste). Par exemple, pour l'hormone oestrogène, on peut citer comme agoniste les régulateurs de l'activité tissulaire oestrogénique (« Selective tissue estrogenic activity regulators» (STEARs) en anglais)), comme la tibolone, et comme antagoniste les modulateurs sélectifs des récepteurs des oestrogènes (« selective estrogen resceptor modulators » (SERMs) en anglais)), tel que le citrate de clomifène, le tamoxifène, le raloxifène, le toremifène ou le lasofoxifène.

Typiquement, dans la formulation susmentionnée :
- l'hormone est choisie parmi :
   - l'oestrogène, notamment choisi parmi l'estrone, l'estriol et l'oestradiol (17-β-oestradiol),
   - la progestérone,
   - l'hydrocortisone,
   - la 5-dehydroepiandrosterone (DHEA),
   - les ganadotrophines, et
   - la kisspeptine,
- l'agoniste d'hormone est :
   - un agoniste d'oestrogène, tel que la tibolone ou un dérivé d'oestrogène choisi parmi les esters d'estrone, d'estriol et d'oestradiol (par exemple l'acétate ou le valérate d'oestradiol), l'estropipate, le cypionate d'estradiol, les estrogènes conjugués équins (CEE) et l'éthinyl oestradiol, ou
   - un progestatif (agoniste de la progestérone), tel que l'acétate de médroxyprogestérone, le noréthistérone, l'acétate de noréthistérone, le lynesténol, le lévonorgestrel, le norgestrel, le norgestimate, le désogestrel, l'étonogestrel, le gestodène, le nogestimate, le norelgestromine, la nandralone, la 17-hydroxyprogestérone ou la drospirénone,
- l'antagoniste d'hormone est :
   - un antiandrogène, tel que l'acétate de cyprotérone, ou
   - un antagoniste des oestrogènes, tel que le citrate de clomifène, le tamoxifène, le raloxifène, le toremifène ou le lasofoxifène.

Les hormones, agonistes et antagonistes susmentionnés sont disponibles dans le commerce ou peuvent être synthétisés par des méthodes connues de l'homme du métier.

Bien entendu, l'hormone, l'agoniste et/ou l'antagoniste de la formulation peut être formulé directement sous sa forme active ou sous forme d'un prodrug.

Les modes de réalisations du tableau 1 suivant sont préférés :

**Tableau 1 : Hormone, agoniste ou antagoniste d'hormone ou mélange de ceux-ci préféré en fonction du type d'hormonothérapie.**

| Type d'hormonothérapie | Hormone, agoniste ou antagoniste d'hormone |
|---|---|
| contraception | - l'oestrogène ou un agoniste d'oestrogène, de préférence la tibolone ou un dérivé d'oestrogène choisi parmi les esters d'estrone, d'estriol et d'oestradiol, l'estropipate, le cypionate d'estradiol, les estrogènes conjugués équins (CEE) et l'éthinyl oestradiol, de préférence la tibolone ou l'éthinyl oestradiol, de préférence l'éthinyl oestradiol, et/ou |
| | - la progestérone ou un progestatif |
| traitement de l'acné | - un antiandrogène, tel que l'acétate de cyprotérone, et/ou |
| | - un progestatif, tel que l'acétate de médroxyprogestérone, la drospirénone ou le norgestimate |
| traitement des ménorragies | la progestérone ou un progestatif |
| traitement de l'infertilité due à une dysrégulation surrénalienne ou aide à la fécondation | Du tamoxifène, du citrate de clomifène, des ganadotrophines, ou de la kisspeptine |
| traitement de la maladie d'Addison | hydrocortisone |
| traitement de la fatigue surrénalienne | l'hydrocortisone ou la 5-dehydroepiandrosterone, et éventuellement de l'acide pantothénique et/ou de la phosphatidylserine |
| traitement hormonal post-ménopause (également appelé traitement hormonal substitutif (THS)) | - l'oestrogène ou un antagoniste d'oestrogène, tel que la tibolone ou un dérivé d'oestrogène choisi parmi les esters d'estrone, d'estriol et d'oestradiol, l'estropipate, le cypionate d'estradiol, les estrogènes conjugués équins (CEE) et l'éthinyl oestradiol, |
| | de préférence la tibolone ou l'éthinyl oestradiol, de préférence l'éthinyl oestradiol, et |
| | - éventuellement de la progestérone ou un progestatif. |

La formulation utilisée comprend le plus souvent une quantité de 0,001 à 30% en poids, généralement moins de 10% en poids, notamment moins de 3% en poids, de préférence entre 0,005 et 1% en poids d'hormone, d'agoniste ou d'antagoniste d'hormone ou de mélange de ceux-ci. En effet, ces proportions sont adaptées pour l'hormonothérapie et, pour ces proportions, la taille des gouttelettes est généralement plus homogène, ce qui permet une libération plus homogène de l'hormone, de l'agoniste ou de l'antagoniste d'hormone ou du mélange de ceux-ci.

On cherche parfois à formuler la nanoémulsion utilisée avec une concentration maximale en hormone, agoniste ou antagoniste d'hormone, notamment lorsqu'ils sont peu solubles, afin de limiter le volume et/ou la durée d'administration au patient.

La formulation utilisée dans l'invention a l'avantage de présenter une très bonne biodisponibilité (bon passage des barrières), cette biodisponibilité étant généralement améliorée par rapport aux systèmes de délivrance existants. Ainsi, plus d'hormone ou d'agoniste ou d'antagoniste d'hormone parvient à la cible, et il est possible de limiter des doses d'hormone ou d'agoniste ou d'antagoniste d'hormone administrée, ce qui est avantageux non seulement en terme de coût, mais surtout car cela permet de limiter les effets secondaires généralement observés lors de l'administration de l'hormone, de l'agoniste d'hormone ou de l'antagoniste d'hormone (à titre d'exemple, les oestrogènes administrés à forte dose favorisent les cancers du sein chez la femme en traitement post-ménopause).

Généralement, l'hormone, l'agoniste et/ou l'antagoniste d'hormone est lipophile et se situe dans la phase dispersée. Il est ainsi encapsulé dans les gouttelettes.

Avantageusement, la formulation utilisée dans l'invention protège l'hormone, l'agoniste et/ou l'antagoniste d'hormone, qui est situé dans les gouttelettes, du corps du patient auquel la formulation est administrée (notamment en évitant son métabolisme prématuré). Cette protection permet également avantageusement de limiter les effets secondaires généralement observés lors de l'administration de l'hormone, de l'agoniste d'hormone ou de l'antagoniste d'hormone.

### ● lipide amphiphile

La formulation utilisée dans l'invention comprend au moins un lipide amphiphile, qui se situe dans la phase dispersée de la nanoémulsion.

Afin de former une nanoémulsion stable, il est généralement nécessaire d'inclure dans la composition au moins un lipide amphiphile à titre de tensioactif. La nature amphiphile du tensioactif assure la stabilisation des gouttelettes d'huile au sein de la phase continue aqueuse.

Les lipides amphiphiles comportent une partie hydrophile et une partie lipophile. Ils sont généralement choisis parmi les composés dont la partie lipophile comprend une chaîne saturée ou insaturée, linéaire ou ramifiée, ayant de 8 à 30 atomes de carbone. Ils peuvent être choisis parmi les phospholipides, les cholestérols, les lysolipides, les sphingomyélines, les tocophérols, les glucolipides, stéarylamines, les cardiolipines d'origine naturelle ou synthétique ; les molécules composées d'un acide gras couplé à un groupement hydrophile par une fonction éther ou ester tels que les esters de sorbitan comme par exemple les monooléate et monolaurate de sorbitan vendus sous les dénominations Span^{®} par la société Sigma; les lipides polymérisés ; les lipides conjugués à de courtes chaînes d'oxyde de polyéthylène (PEG) tels que les tensioactifs non-ioniques vendus sous les dénominations commerciales Tween^{®} par la société ICI Americas, Inc. et Triton^{®} par la société Union Carbide Corp.; les esters de sucre tels que les mono- et di-laurate, mono- et di-palmitate, mono- et distéarate de saccharose; lesdits tensioactifs pouvant être utilisés seuls ou en mélanges.

Les phospholipides sont les lipides amphiphiles préférés.

La lécithine est le lipide amphiphile particulièrement préféré.

Généralement, la phase dispersée comportera de 0,01 à 99% en poids, de préférence de 5 à 75% en poids, en particulier de 5 à 50% et tout particulièrement de 8 à 30% en poids de lipide amphiphile.

La quantité de lipide amphiphile contribue avantageusement à contrôler la taille de la phase dispersée de la nanoémulsion.

### ● lipide solubilisant

La formulation utilisée dans l'invention comprend par ailleurs un lipide solubilisant, qui se situe dans la phase dispersée de la nanoémulsion. Ce composé a pour mission principale de solubiliser le lipide amphiphile, peu soluble, dans la phase huileuse de la nanoémulsion.

Le lipide solubilisant est un lipide présentant une affinité avec le lipide amphiphile suffisante pour permettre sa solubilisation. De préférence, le lipide solubilisant est solide à température ambiante.

Dans le cas où le lipide amphiphile est un phospholipide, il peut s'agir notamment de dérivés du glycérol, et en particulier de glycérides obtenues par estérification de glycérol avec des acides gras.

Le lipide solubilisant utilisé est avantageusement choisi en fonction du lipide amphiphile utilisé. Il présentera généralement une structure chimique proche, afin d'assurer la solubilisation recherchée. Il peut s'agir d'une huile ou d'une cire. De préférence, le lipide solubilisant est solide à température ambiante (20 °C), mais liquide à la température du corps (37°C).

Les lipides solubilisants préférés, en particulier pour les phospholipides, sont les glycérides d'acides gras, notamment d'acides gras saturés, et en particulier d'acides gras saturés comportant 8 à 18 atomes de carbone, encore préféré 12 à 18 atomes de carbone. Avantageusement, il s'agit d'un mélange de différents glycérides.

De préférence, il s'agit de glycérides d'acides gras saturés comportant au moins 10% en poids d'acides gras en C12, au moins 5% en poids d'acides gras en C14, au moins 5% en poids d'acides gras en C16 et au moins 5% en poids d'acides gras en C18.

De préférence, il s'agit de glycérides d'acides gras saturés comportant 0% à 20% en poids d'acides gras en C8, 0% à 20% en poids d'acides gras en C10, 10% à 70% en poids d'acides gras en C12, 5% à 30% en poids d'acides gras en C14, 5% à 30% en poids d'acides gras en C16 et 5% à 30% en poids d'acides gras en C18.

Particulièrement préférés sont les mélanges de glycérides semi-synthétiques solides à température ambiante vendus sous la dénomination commerciale Suppocire^{®}NC par la société Gattefossé et approuvé pour l'injection chez l'homme. Les Suppocire^{®} de type N sont obtenues par estérification directe d'acides gras et de glycérol. Il s'agit de glycérides hémi-synthétiques d'acides gras saturés de C8 à C18, dont la composition quali-quantitative est indiquée dans le tableau ci-dessous.

Les lipides solubilisants précités permettent d'obtenir une formulation sous forme de nanoémulsion avantageusement stable. Sans vouloir être lié à une théorie particulière, il est supposé que les lipides solubilisants précités permettent d'obtenir des gouttelettes dans la nanoémulsion présentant un coeur amorphe. Le coeur ainsi obtenu présente une viscosité interne élevée sans pour autant présenter de cristallinité. Or, la cristallisation est néfaste pour la stabilité de la nanoémulsion car elle conduit généralement à une agrégation des gouttelettes et/ou à une expulsion des molécules encapsulées à l'extérieur des gouttelettes. Ces propriétés physiques favorisent la stabilité physique de la nanoémulsion et la stabilité de l'encapsulation au cours du temps de l'hormone, de l'antagoniste ou de l'agoniste d'hormone ou de leur mélange.

La quantité de lipide solubilisant peut varier largement en fonction de la nature et de la quantité de lipide amphiphile présent dans la phase huileuse. Généralement, la phase huileuse comportera de 1 à 99% en poids, de préférence de 5 à 80% en poids et tout particulièrement de 40 à 75% en poids de lipide solubilisant.

### Composition en acides gras du Suppocire^{®} NC de Gattefossé

| Longueur de chaînes | [% en poids] |
|---|---|
| C8 | 0,1 à 0,9 |
| C10 | 0,1 à 0,9 |
| C12 | 25 à 50 |
| C14 | 10 à 24,9 |
| C16 | 10 à 24,9 |
| C18 | 10 à 24,9 |

### ● huile

La phase huileuse peut comporter par ailleurs une ou plusieurs autres huiles, qui se situe(nt) dans la phase dispersée.

Les huiles utilisées présentent de préférence une balance hydrophile-lipophile (HLB) inférieure à 8 et encore plus préférentiellement comprise entre 3 et 6. Avantageusement, les huiles sont utilisées sans modification chimique ou physique préalablement à la formation de l'émulsion.

Les huiles sont généralement choisies parmi les huiles biocompatibles, et en particulier parmi les huiles d'origine naturelle (végétale ou animale) ou synthétique. Parmi de telles huiles, on peut notamment citer les huiles d'origine naturelle végétale parmi lesquelles figurent notamment les huiles de soja, de lin, de palme, d'arachide, d'olives, de pépin de raisins et de tournesol ; les huiles synthétiques parmi lesquelles figurent notamment les triglycérides, di glycérides et les mono glycérides. Ces huiles peuvent être de premières expressions, raffinées ou inter-estérifiées.

Les huiles préférées sont l'huile de soja et l'huile de lin.

Généralement, si présente, l'huile sera contenue dans la phase huileuse dans une proportion allant de 1 à 80% en poids, de préférence entre 5 et 50 % en poids et tout particulièrement 10 à 30% en poids.

La phase huileuse peut contenir en outre d'autres additifs tels que des colorants, stabilisants, conservateurs ou d'autres principes actifs, en quantité appropriée.

### ● Co-tensioactif

La formulation utilisée dans l'invention comprend généralement un co-tensioactif, qui permet de stabiliser la nanoémulsion.

Le co-tensioactif peut par ailleurs présenter d'autres effets dans l'application envisagée de la nanoémulsion.

Les co-tensioactifs utilisables dans les formulations utilisées dans l'invention sont de préférence des tensioactifs hydrosolubles. Les tensioactifs hydrosolubles sont de préférence alkoxylés (ils comprennent au moins une chaîne composée de motifs d'oxyde d'alkylène) et comportent de préférence au moins une chaîne composée de motifs d'oxyde d'éthylène (PEO ou PEG) ou d'oxyde d'éthylène et d'oxyde de propylène. De préférence, le nombre de motifs dans la chaîne varie entre 2 et 500.

A titre d'exemple de co-tensioactifs, on peut en particulier citer les composés conjugués polyéthylèneglycol/phosphatidyl-éthanolamine (PEG-PE), les éthers d'acide gras et de polyéthylèneglycol tels que les produits vendus sous les dénominations commerciales Brij^{®} (par exemple Brij^{®} 35, 58, 78 ou 98) par la société ICI Americas Inc., les esters d'acide gras et de polyéthylèneglycol tels que les produits vendus sous les dénominations commerciales Myrj^{®} par la société ICI Americas Inc. (par exemple Myrj^{®} 45, 52, 53 ou 59) et les copolymères blocs d'oxyde d'éthylène et d'oxyde de propylène tels que les produits vendus sous les dénominations commerciales Pluronic^{®} par la société BASF AG (par exemple Pluronic^{®} F68, F127, L64, L61, 10R4, 17R2, 17R4, 25R2 ou 25R4 ) ou les produits vendus sous la dénomination commerciale Synperonic^{®} par la société Unichema Chemie BV (par exemple Synperonic^{®} PE/F68, PE/L61 ou PE/L64).

Ainsi, le co-tensioactif se situe à la fois dans la phase aqueuse continue et dans la phase dispersée. En effet, la partie hydrophobe du co-tensioactif s'insère dans les gouttelettes de la phase dispersée, alors que les chaînes polyalcoxylées sont dans la phase aqueuse continue. Dans la présente demande, les pourcentages massiques de phase dispersée décrits sont calculés en considérant que le co-tensioactif appartient à la phase dispersée.

La phase aqueuse comporte de 0,01 à 50% en poids, de préférence de 1 à 30% en poids et tout particulièrement de 4 à 20% en poids de co-tensioactif.

Selon un mode de réalisation, la phase dispersée de la nanoémulsion est greffée en surface avec des molécules d'intérêts tels que des ligands biologiques, afin d'augmenter le ciblage spécifique envers les organes stéroïdiens, ou pour cibler un autre organe. Un tel greffage permet une reconnaissance spécifique de certaines cellules ou de certains organes. De préférence, le greffage en surface est réalisé par couplage des molécules d'intérêts ou de leurs précurseurs avec un composé amphiphile, notamment avec le co-tensioactif. La nanoémulsion comprend alors un co-tensioactif greffé. Dans ce cas, le co-tensioactif joue le rôle d'un espaceur permettant d'accommoder les molécules d'intérêt en surface.

Les molécules d'intérêt peuvent être par exemple :
- des ligands biologiques de ciblage tels que des anticorps, peptides, saccharides, aptamères, oligonucléotides ou des composés comme l'acide folique ;
- un agent de furtivité : une entité ajoutée afin de conférer à la nanoémulsion une furtivité vis-à-vis du système immunitaire, d'augmenter son temps de circulation dans l'organisme, et de ralentir son élimination.

Par exemple, lorsque le ligand biologique est un peptide comprenant une ou plusieurs cystéine, le greffage à la chaîne oxyde d'alkylène du tensioactif peut être assuré par couplage thiol maléimide.

### ● agent d'imagerie

Dans un mode de réalisation, la formulation utilisée comprend un agent d'imagerie, qui permet avantageusement de visualiser la distribution des gouttelettes dans le corps du patient, et donc la distribution de l'hormone et/ou de l'agoniste ou de l'antagoniste d'hormone.

L'agent d'imagerie peut notamment être utilisé dans les imageries de type :
- tomographie à émission de positons (Positron Emission Tomography (PET) en anglais) (l'agent d'imagerie pouvant être un composé comprenant un radionucléide, tel que ¹⁸F, ¹¹C, un chélate de cations métalliques ⁶⁸Ga, ⁶⁴Cu),
- tomographie d'émission monophotonique (TEMP) (Single photon emission computed tomography (SPECT) en anglais) (l'agent d'imagerie pouvant être un composé comprenant un radionucléide par exemple ¹²³I, ou un chélate de ^{99m}Tc ou ¹¹¹In),
- imagerie par résonance magnétique (IRM) (l'agent d'imagerie pouvant être un chélate de gadolinium ou un nanocristal magnétique tel qu'un nanocristal d'oxyde de fer, d'oxyde de manganèse ou de fer-platine FePt),
- imagerie optique ou imagerie aux rayons X (l'agent d'imagerie pouvant être un fluorophore lipophile ou un agent de contraste, par exemple une molécule iodée telle que l'iopamidol, l'amidotrizoate, ou des nanoparticules d'or).

De préférence, l'agent d'imagerie est un fluorophore lipophile permettant de réaliser de l'imagerie optique.

La nature du ou des fluorophores lipophiles utilisables n'est pas critique à partir du moment où ils sont compatibles avec l'imagerie in vivo (c'est à dire qu'ils sont biocompatibles et non toxiques). De préférence, les fluorophores utilisés comme agent d'imagerie absorbent et émettent dans le visible et le proche infrarouge, en particulier dans le proche infrarouge. En effet, pour que la lumière d'excitation et la lumière émise par le fluorophore puissent mieux traverser les tissus, il convient d'utiliser des fluorophores absorbant et émettant dans le proche infrarouge, c'est-à-dire à une longueur d'onde comprise entre 640 et 900 nm.

A titre de fluorophore lipophile on peut par exemple citer les composés décrits dans le chapitre 13 ("Probes for Lipids and Membranes") du catalogue InVitrogen. De façon plus précise, on peut notamment citer, à titre de fluorophore, le vert d'indocyanine (ICG), les analogues d'acides gras et les phospholipides fonctionnalisés par un groupement fluorescent tels que les produits fluorescents vendus sous les dénominations commerciales Bodipy (R) tels que le Bodipy (R) 665/676 (Ex/Em.) ; les dérivés amphiphiles de dialkylcarbocyanines telles que le perchlorate de I,r-dioctadécyl-3,3,3',3'-tétraméthylindodicarbocyanine (DiD), par exemple vendu sous la référence D-307 ; les sondes fluorescentes dérivées de sphingolipides, de stéroïdes ou de lipopolysaccharides tels que les produits vendus sous les dénominations commerciales BODIPY ® TR céramides, BODIPY ® FL C5-lactosylcéramide, BODIPY ® FL C5-ganglioside, BODIPY ® FL cérébrosides ; les dérivés amphiphiles de cyanines, de rhodamines, de fluorescéines ou de coumarines tels que l'octadécyl rhodamine B, l'octadécyl ester de fluorescéine et la 4-heptadécyl-7- hydroxycoumarine ; et le diphénylhexatriène (DPH) et ses dérivés ; l'ensemble de ces produits étant vendus par la société Invitrogen.

Selon une forme de réalisation préférée de l'Invention, le fluorophore est le vert d'indocyanine ou le perchlorate de I,r-dioctadécyl-3,3,3',3'-tétraméthylindodicarbocyanine.

### ● Phase aqueuse

La phase aqueuse de la nanoémulsion utilisée dans l'invention est de préférence constituée d'eau et/ou d'un tampon tel qu'un tampon phosphate comme par exemple du PBS ("Phosphate Buffer Saline") ou d'une solution saline, notamment de chlorure de sodium.

Selon un mode de réalisation, la phase aqueuse continue comporte également un agent épaississant tel que du glycérol, un saccharide, oligosaccharide ou polysaccharide, une gomme ou encore une protéine, de préférence du glycérol. En effet, l'utilisation d'une phase continue de viscosité plus élevée facilite l'émulsification et permet de ce fait de réduire le temps de sonication.

La phase aqueuse comporte avantageusement de 0 à 50% en poids, de préférence de 1 à 30% en poids et tout particulièrement de 5 à 20% en poids d'agent épaississant.

Bien entendu, la phase aqueuse peut contenir en outre d'autres additifs tels que des colorants, stabilisants et conservateurs en quantité appropriée.

La proportion de phase dispersée et de phase aqueuse est très variable. Cependant, le plus souvent, les nanoémulsions seront préparées avec 1 à 50%, de préférence 5 à 40% et tout particulièrement 10 à 30% en poids de phase dispersée (i.e. l'ensemble (éventuelle huile/lipide solubilisant/lipide amphiphile/co-tensioactif/éventuel agent d'imagerie)) et 50 à 99%, de préférence 60 à 95% et tout particulièrement 70 à 90 % en poids de phase aqueuse.

### ● Nanoémulsion sous forme de gel

Dans un mode de réalisation, la phase dispersée de la formulation utilisée représente entre 35 et 65% en poids, de préférence entre 45 et 64% en poids par rapport au poids total de la nanoémulsion.

Dans ce mode de réalisation, la nanoémulsion utilisée se présente sous forme de « gel ». On entend par le terme « gel » habituellement un système biphasique solide-liquide thermodynamiquement stable, constitué d'un double réseau interpénétré continu tridimensionnel, l'un solide et le second liquide. Un tel gel est un système biphasique liquide-solide dont le réseau solide retient une phase liquide. Bien que les gels puissent être considérés comme solides, ils présentent des propriétés propres aux solides (rigidité structurelle, élasticité à la déformation) comme aux liquides (pression de vapeur, de compressibilité et conductivité électrique).

Dans le cas d'une nanoémulsion sous forme de gel, le réseau tridimensionnel est formé par les gouttelettes, les interstices entre gouttelettes étant remplis de phase continue. Les liaisons entre les unités du réseau, à savoir les gouttelettes, reposent généralement sur des interactions non covalentes de type liaison hydrogène, interactions de Van der Waals ou encore interactions électrostatiques (paires d'ions). Ces interactions existent principalement entre les co-tensioactifs de gouttelettes adjacentes.

Une nanoémulsion sous forme de gel montre donc une résistance à la pression et est capable de maintenir une forme définie, ce qui peut être avantageux selon la forme et/ou la voie d'administration souhaitée.

Pour mettre en évidence que la nanoémulsion est sous forme de gel, on peut réaliser des études rhéologiques permettant d'évaluer les propriétés viscoélastiques, et/ou des études plus structurelles montrant les liaisons entre les gouttelettes formant le réseau tridimensionnel (diffraction aux rayons X, neutrons...). En effet, une nanoémulsion sous forme de gel possède une viscosité et un coefficient d'élasticité plus important qu'une nanoémulsion liquide. La nanoémulsion sous forme de gel peut, en fonction de la concentration de gouttelettes et donc de la fraction massique en phase dispersée, se trouver à l'état de liquide visqueux, de solide viscoélastique ou de solide élastique. Par rapport à la phase dispersante aqueuse, dont la viscosité est proche de celle de l'eau (1 mPa.s à 25°C), la nanoémulsion est considérée comme un liquide visqueux lorsque sa viscosité est 10 fois plus élevée que celle de l'eau, soit > 10 mPa.s à 25°C. Par ailleurs, lorsque l'on procède à la mesure rhéologique des modules de G' (module de conservation en cisaillement) et G" (module de perte en cisaillement), on considère que la nanoémulsion est sous forme d'un liquide visqueux lorsque G" >G'. Lorsque G' devient proche de G", la nanoémulsion est à l'état de solide viscoélastique. Lorsque G" < G', on est à l'état de solide élastique. Dans ce mode de réalisation, la nanoémulsion se présente de préférence à l'état liquide visqueux ou de solide viscoélastique, car la viscosité est suffisamment modérée dans ces états pour permettre des applications impliquant une administration par injection. La viscosité et le coefficient d'élasticité peuvent être mesurés par un rhéomètre cône-plan ou par un rhéomètre Couette. La viscosité d'une nanoémulsion liquide est généralement inférieure à 1 poise, voire même souvent inférieure à 0,01 poise. La nanoémulsion utilisée dans ce mode de réalisation de l'invention a généralement une viscosité supérieure à 1 poise, et pourra avoir une viscosité allant jusqu'à celle d'un solide (plus de 1000 poises). La nanoémulsion de la présente invention a généralement une viscosité de 1 à 1000 poises, préférentiellement de 1 à 500 poises et encore plus préférentiellement entre 1 et 200, ces valeurs étant données à 25°C. Une viscosité supérieure à 1 poise est en effet adaptée pour que les gouttelettes de la phase dispersée forment un réseau tridimensionnel à l'intérieur de la phase continue. En effet, il a été constaté que en dessous de 1 poise, les gouttelettes ne sont généralement pas assez proches les unes des autres,. Au dessus de 1000 poises, on obtient un système quasi-solide. La nanoémulsion est alors trop visqueuse ce qui rend son utilisation difficile. De même, alors que le coefficient d'élasticité est généralement inférieur à 10 dans le cas d'une nanoémulsion liquide, le coefficient d'élasticité d'une nanoémulsion sous forme de gel est généralement supérieur à 10. Les études structurelles, notamment les diffractions aux rayons X ou aux neutrons, permettent également de différencier l'organisation d'une nanoémulsion liquide, de l'organisation d'une nanoémulsion sous forme de gel. En effet, les pics du diffractogramme obtenu pour une nanoémulsion liquide sont caractéristiques de la structure des gouttelettes de phase dispersée (grand angles de diffraction caractéristiques de distances courtes), alors que les pics du diffractogramme d'une nanoémulsion sous forme de gel sont caractéristiques non seulement de la structure des gouttelettes (grand angles de diffraction caractéristiques de distances courtes) mais aussi de l'organisation de ces gouttelettes en réseau tridimensionnel (faibles angles de diffraction caractéristiques de distances plus grandes).

La nanoémulsion utilisée dans ce mode de réalisation de l'invention est avantageusement sous forme de gel dispersible, c'est-à-dire que les gouttelettes formant le réseau tridimensionnel peuvent être relarguées dans la phase continue sous certaines conditions par « dégélification » du système gel, également dénommée « désagrégation » dans la présente demande. La désagrégation est observée par ajout de phase continue au gel, par mise en contact avec les fluides physiologiques lors de l'administration de la nanoémulsion ou par augmentation de la température. En effet, ajouter de la phase continue entraîne une différence de pression osmotique entre l'intérieur du gel et la phase continue. Le système tendra donc à diminuer, jusqu'à annuler, cette différence de pression osmotique en libérant les gouttelettes dans l'excès de phase continue, jusqu'à obtenir une concentration en gouttelettes homogène dans l'ensemble du volume de phase continue. De même augmenter suffisamment la température du système revient à donner aux différentes gouttelettes une énergie thermique supérieure aux énergies mises en jeu dans les liaisons, par exemple les liaisons hydrogène, et ainsi à rompre ces liaisons et libérer les gouttelettes du réseau tridimensionnel. Ces températures dépendent de la composition du gel et plus particulièrement de la taille des gouttelettes et de la longueur des chaînes polyalcoxylées du co-tensioactif. La désagrégation de la nanoémulsion sous forme de gel peut être suivie par diffraction aux rayons X, par calorimétrie différentielle à balayage (DSC) ou par résonance magnétique nucléaire (RMN). En suivant par diffraction aux rayons X la désagrégation de la nanoémulsion sous forme de gel, on observe une évolution du spectrogramme, c'est-à-dire une diminution de l'intensité des angles faibles (caractéristiques de l'organisation des gouttelettes en réseau tridimensionnel) (comme décrit dans Matija Tomsic, Florian Prossnigg, Otto Glatter 'Journal of Colloid and Interface Science' Volume 322, Issue 1, 1 June 2008, Pages 41-50). La désagrégation peut également être suivie par DSC. Un pic apparaît sur le thermogramme lors de la transition nanoémulsion sous forme de gel / nanoémulsion liquide en montée en température. Enfin, une étude RMN peut aussi permettre de suivre la désagrégation par mesure du coefficient de diffusion associé à chaque gouttelette en distinguant une nanoémulsion liquide d'une nanoémulsion sous forme de gel. En effet, le coefficient de diffusion est très significativement diminué dans le cas d'une nanoémulsion sous forme de gel (il est alors généralement inférieur à 0.01µm²/s), où le système est figé. (WESTRIN B. A.; AXELSSON A.; ZACCHI G. 'Diffusion measurement in gels', Journal of controlled release 1994, vol. 30, n°3, pp. 189-199).

### ● Emulsion dans laquelle les gouttelettes sont liées entre-elles de façon covalente

Dans un mode de réalisation, la formulation utilisée comprend un tensioactif de formule (I) suivante :

(L₁-X₁-H₁-Y₁)ᵥ-G-(Y₂-H₂-X₂-L₂)_{w} (I),

dans laquelle :
- L₁ et L₂ représentent indépendamment des groupes lipophiles,
- X₁, X₂, Y₁, Y₂ et G représentent indépendamment un groupe de liaison,
- H₁ et H₂ représentent indépendamment des groupes hydrophiles comprenant une chaîne polyalcoxylée,
- v et w sont indépendamment un nombre entier de 1 à 8,
et les gouttelettes de la phase dispersée sont liées entre-elles de façon covalente par le tensioactif de formule (I).

Le tensioactif de formule (I) se situe partiellement dans la phase aqueuse continue et partiellement dans la phase dispersée. En effet, le tensioactif de formule (I) comprend deux groupes lipophiles (L₁ et L₂) et deux groupes hydrophiles (H₁ et H₂). Les groupes hydrophiles sont situés majoritairement à la surface des gouttelettes, dans la phase aqueuse continue alors que les groupes lipophiles se situent dans les gouttelettes de la formulation.

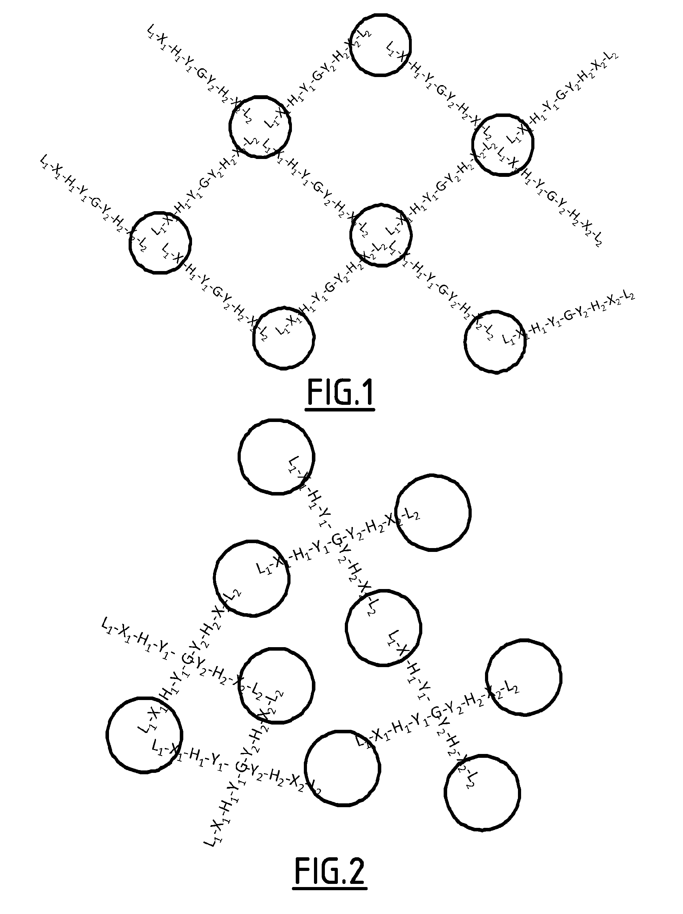

Plus précisément, le groupe lipophile L₁ se situe dans certaines gouttelettes, et le groupe L₂ dans des gouttelettes adjacentes. Les gouttelettes sont donc reliées de façon covalente entre-elles par le groupe -(X₁-H₁-Y₁)ᵥ-G-(Y₂-H₂₋X₂)_{w}- du tensioactif de formule (I). Les figures 1 et 2 illustrent le positionnement du tensioactif de formule (I) respectivement lorsque v et w représentent 1 et lorsque v et w représentent 2.

Les groupes X₁ et X₂ sont des groupes de liaison liant les groupes lipophile et hydrophobe. Le groupe G est un groupe de liaison entre les deux parties [lipophile-hydrophile] du tensioactif de formule (I). Les groupes Y₁ et Y₂ sont des groupes de liaison liant le groupe G à ces deux parties [lipophile-hydrophile].

La formulation utilisée dans ce mode de réalisation peut avantageusement être mise en forme (par exemple en la plaçant dans un moule ou un récipient ayant une forme donnée), et rester dans la forme désirée selon l'application souhaitée. Ce mode de réalisation de l'invention est donc particulièrement adapté lorsque la formulation est utilisée sous forme de gélule, de gel, d'ovule ou de patch.

D'autre part, elle résiste à la dilution à une phase aqueuse. Plus précisément, lorsqu'une phase aqueuse est ajoutée à cette formulation, celle-ci reste en forme et ne se dilue pas. Dans le milieu, on observe d'une part la formulation comprenant les gouttelettes, et d'autre part une phase aqueuse essentiellement exempte de gouttelettes.

Sans vouloir être liés à une théorie particulière, il semble que ces propriétés de cette formulation puissent être expliquées par la présence des liaisons covalentes entre les gouttelettes, qui confèrent une cohésion très forte entre elles.

Dans un mode de réalisation, dans la formule (I) susmentionnée :
- L₁ et L₂ sont indépendamment choisis parmi :
   - un groupe R ou R-(C=O)-, où R représente une chaîne hydrocarbonée linéaire comprenant de 11 à 23 atomes de carbone,
   - un ester ou un amide d'acides gras comprenant de 12 à 24 atomes de carbone et de phosphatidyléthanolamine, tel que le distéaryl phosphatidyléthanolamine (DSPE), et
   - un poly(oxyde de propylène), et/ou
- X₁, X₂, Y₁ et Y₂ sont indépendamment choisis parmi :
   - une liaison simple,
   - un groupe Z choisi parmi -O-, -NH-, -O(OC)-, -(CO)O-, -(CO)NH-, -NH(CO)-, -O-(CO)-O-, -NH-(CO)-O-, -O-(CO)-NH- et -NH-(CO)-NH,
   - un groupe Alk étant un alkylène comprenant de 1 à 6 atomes de carbone, et
   - un groupe Z-Alk, Alk-Z, Alk-Z-Alk ou Z-Alk-Z où Alk et Z sont tels que définis ci-dessus et où les deux groupes Z du groupe Z-Alk-Z sont identiques ou différents, et/ou
- H₁ et H₂ sont indépendamment choisis parmi un poly(oxyde d'éthylène) comprenant typiquement de 3 à 500 unités oxyde d'éthylène, de préférence de 20 à 200 unités oxyde d'éthylène, et/ou
- G comprend au moins un groupe G' ayant l'une des formules suivantes (les groupes Y₁ et Y₂ étant reliés à gauche et à droite des formules décrites ci-dessous): 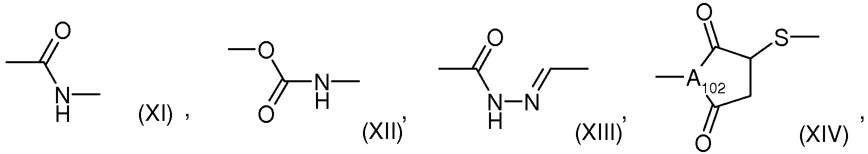 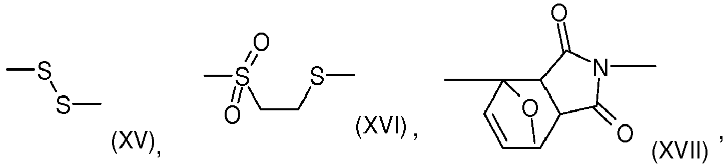 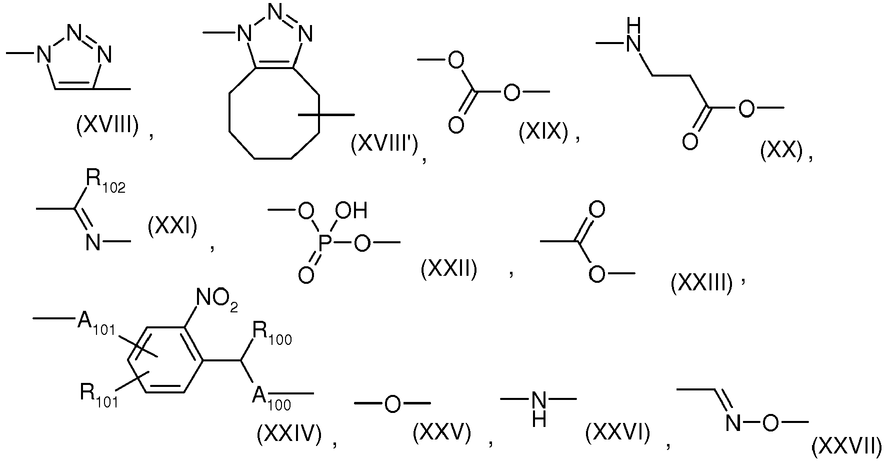 où A₁₀₂ représente CH ou N, R₁₀₂ représente H ou une chaîne hydrocarbonée linéaire comprenant de 1 à 6 atomes de carbone, A₁₀₁ représente -O-, -NH-(CO)- ou -O-(CO)-, R₁₀₀ représente H ou un méthyle, A₁₀₀ représente -O- ou -NH- et R₁₀₁ représente H, Me ou -OMe.

Par la formule 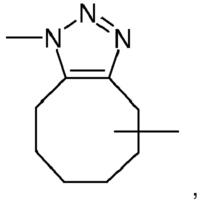 on entend que le groupe Y₂ peut être relié à n'importe lequel des six atomes du cyclooctyle et par la formule 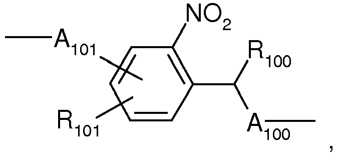 on entend que les groupe A₁₀₁ et R₁₀₁ peuvent être relié à n'importe lequel des quatre atomes du phényle.

Notamment, v et w représentent indépendamment 1 ou 2. v et w représentent de préférence 1.

Le groupe G peut comprendre un ou plusieurs des groupes G' définis ci-dessus.

Ainsi, dans un premier mode de réalisation, le groupe G est constitué d'un groupe G'. Dans ce mode de réalisation, dans la formule (I), v et w représentent 1.

Dans un second mode de réalisation, le groupe G répond à la formule -G'-Y₃-G'- dans laquelle :
- Y₃ représente un groupe de liaison, notamment choisi parmi :
   - une liaison simple,
   - un groupe Z choisi parmi -O-, -NH-, -O(OC)-, -(CO)O-, -(CO)NH-, -NH(CO)-, -O-(CO)-O-, -NH-(CO)-O-, -O-(CO)-NH- et -NH-(CO)-NH,
   - un groupe Alk étant un alkylène comprenant de 1 à 6 atomes de carbone, et
   - un groupe Z-Alk, Alk-Z, Alk-Z-Alk ou Z-Alk-Z où Alk et Z sont tels que définis ci-dessus et où les deux groupes Z du groupe Z-Alk-Z sont identiques ou différents
- chacun des G' représente indépendamment un groupe de formule (XI) à (XXVI) susmentionnés, et de préférence, les deux groupes G' de la formule -G'-Y3-G'- sont identiques.

Dans ce mode de réalisation, dans la formule (I), v et w représentent 1.

Ce mode de réalisation est particulièrement intéressant lorsque les deux groupes G' sont identiques et comprennent une fonction clivable. En effet, il suffit alors de cliver une seule des deux fonctions pour rompre les liaisons covalentes entre les gouttelettes de la formulation.

Dans un troisième mode de réalisation, le groupe G est un dendrimère comprenant (v+w) groupes G'. Le groupe G peut notamment être un dendrimère comprenant plusieurs groupes G', tel qu'un dendrimère comprenant un groupe polyamidoamine (PAMAM). Par exemple, le groupe G peut avoir l'une des formules (XXX) à (XXXIII) suivantes, qui comprennent :
- 4 groupes G' de formule (XXVI). v et w représentent 2.
- 4 groupes G' de formule (XXIV) où R₁₀₁ représente -O-Me, A₁₀₁ représente -NH-, R₁₀₀ représente un méthyle et A₁₀₀ représente -NH-. v et w représentent 2.
- 4 groupes G' de formule (XIV). v et w représentent 2.
- 16 groupes G' de formule (XXVI). v et w représentent 8.

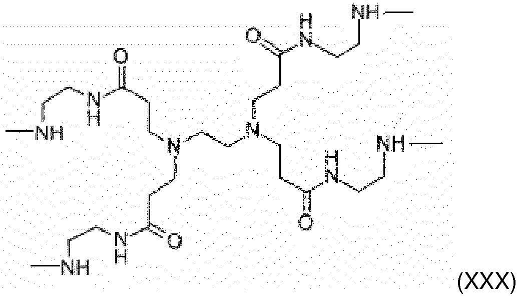

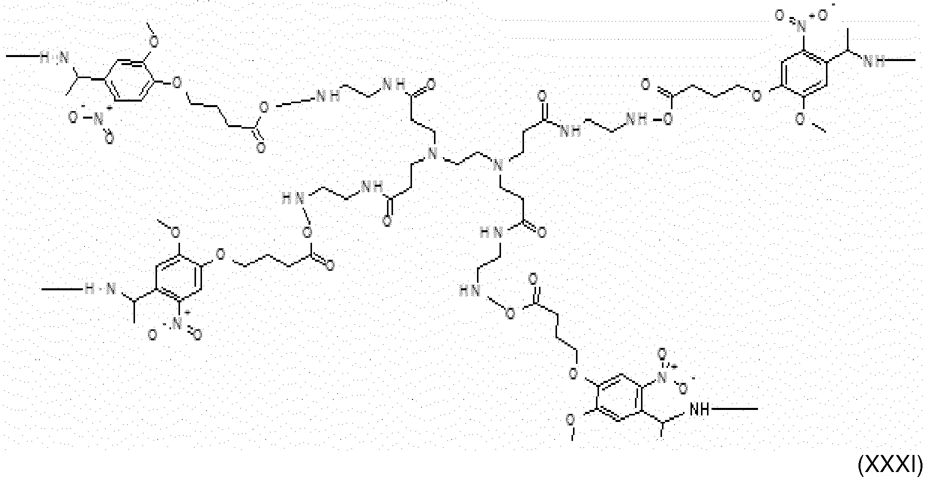

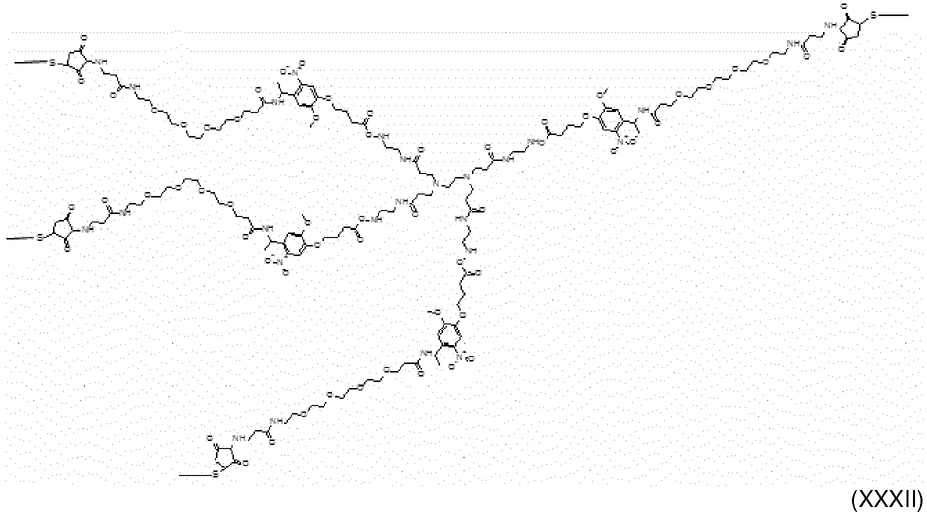

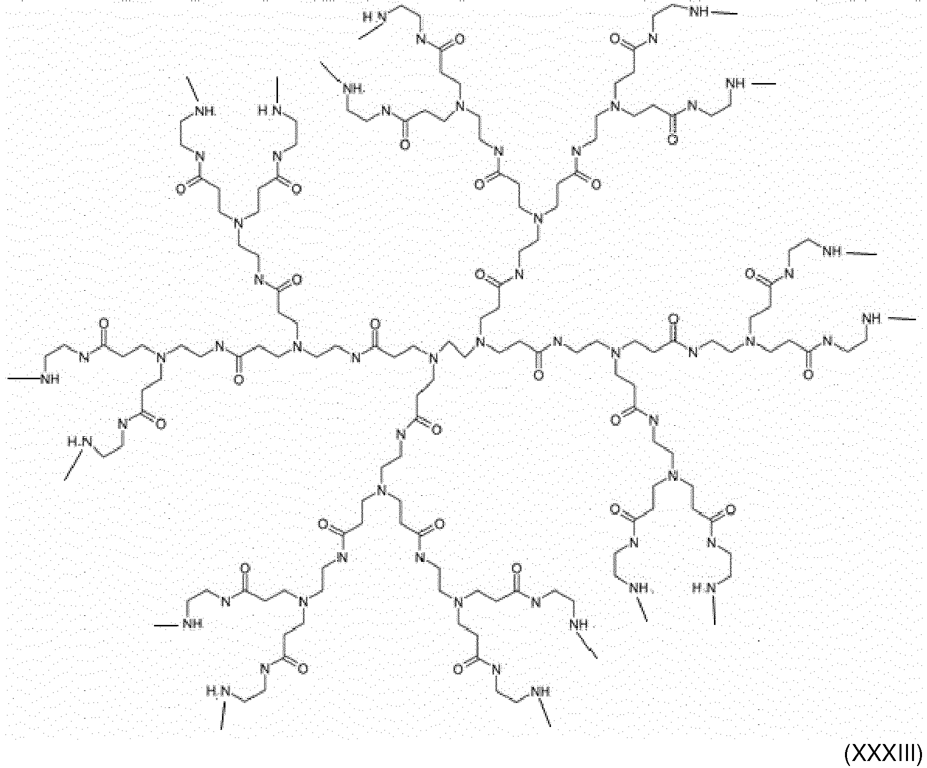

Lorsque L₁ et/ou L₂ représentent un groupe R-(C=O)-, où R représente une chaîne hydrocarbonée linéaire comprenant de 11 à 23 atomes de carbone, L₁ et/ou L₂ représentent des groupes issus d'acide gras comprenant de 12 à 24 atomes de carbone.

Par «L₁ et L₂ représentent un ester ou un amide d'acides gras comprenant de 12 à 24 atomes de carbone et de phosphatidyléthanolamine», on entend qu'ils représentent un groupe de formule : 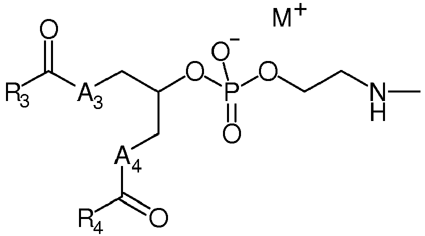 dans laquelle
- R₃ et R₄ représentent indépendamment une chaîne hydrocarbonée linéaire comprenant de 11 à 23 atomes de carbone,
- A₃ et A₄ représentent O ou NH, et
- M représente H ou un cation.

De préférence, L₁ et L₂ sont identiques et/ou X₁ et X₂ sont identiques et/ou H₁ et H₂ sont identiques. Des tensioactifs de formule (I) particulièrement préférés sont ceux dans lesquels L₁ et L₂ sont identiques, X₁ et X₂ sont identiques et.H₁ et H₂ sont identiques. Ces tensioactifs sont en effet des composés symétriques et sont donc généralement plus faciles à synthétiser, et donc moins coûteux.

Dans un mode de réalisation, dans la formule (I) susmentionnée, les radicaux L₁-X₁-H₁- et/ou L₂-X₂-H₂- consistent en l'un des groupes de formules suivantes (le groupe Y₁ ou Y₂ étant relié à droite des formules décrites ci-dessous): 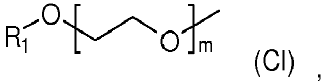 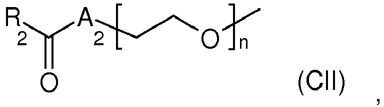 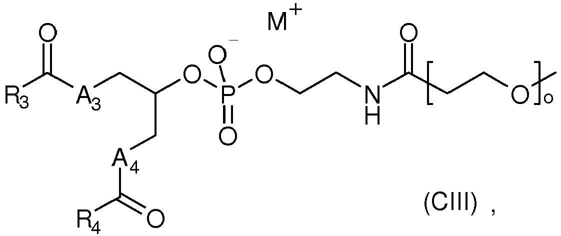 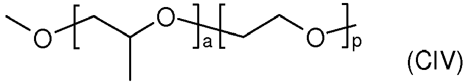 dans lesquelles :
- R₁, R₂, R₃ et R₄ représentent indépendamment une chaîne hydrocarbonée linéaire comprenant de 11 à 23 atomes de carbone,
- A₁, A₂, A₃ et A₄ représentent O ou NH,
- m, n, o et p représentent indépendamment des nombres entiers de 3 à 500, de préférence 20 à 200, et
- a représente un nombre entier de 20 à 120,
- M représente H ou un cation.

Les radicaux L₁-X₁-H₁-et/ou L₂-X₂-H₂- de formule (CII) sont préférés. En effet, ils sont faciles à préparer (notamment par formation d'un ester ou d'une amide entre un acide gras et un dérivé de poly(éthylène glycol). De plus, une formulation comprenant un tensioactif comprenant un radical L₁-X₁-H₁-et/ou L₂-X₂-H₂- de formule (CII) peut généralement être préparé avec une quantité plus importante de ce tensioactif qu'une formulation comprenant un tensioactif comprenant un radical L₁-X₁-H₁-et/ou L₂-X₂-H₂- de formule (CIII). Or, plus la proportion de tensioactif de formule (I) dans la formulation est élevée, plus la cohésion entre les gouttelettes est grande, et plus la formulation reste en forme et résiste à la dilution. Ainsi, ces deux propriétés peuvent être encore exacerbées pour une formulation comprenant un tensioactif comprenant un radical L₁-X₁-H₁-et/ou L₂-X₂-H₂- de formule (CII).

Les radicaux L₁-X₁-H₁-et/ou L₂-X₂-H₂- de formule (CII) avec A₂ représente NH sont particulièrement préférés, car les tensioactifs comprenant de tels radicaux permettent d'éviter la fuite des agents d'intérêts lipophiles éventuellement présents hors des gouttelettes de la formulation plus efficacement que des tensioactifs comprenant des radicaux L₁-X₁-H₁-et/ou L₂-X₂-H₂- de formule (CII) avec A₂ représente O.

Dans un mode de réalisation, dans la formule (I), v et w représentent 1, L₁ et L₂ sont indépendamment R-(C=O)-, où R représente une chaîne hydrocarbonée linéaire comprenant de 11 à 23 atomes de carbone, H₁ et H₂ sont indépendamment des poly(oxyde d'éthylène) comprenant de 3 à 500 unités oxyde d'éthylène, X₁ et X₂ représentent -O- ou -NH-, G est constitué d'un groupe G' représentant -S-S- (groupe de formule (XV) ci-dessus) et Y₁ et Y₂ représentent -CH₂-CH₂-NH-CO-CH₂-CH₂- (groupe Alk-Z-Alk ci-dessus avec Alk représente -CH₂-CH₂- et Z représente -NH-(CO)-) et le tensioactif de la formulation a alors la formule (I') suivante : 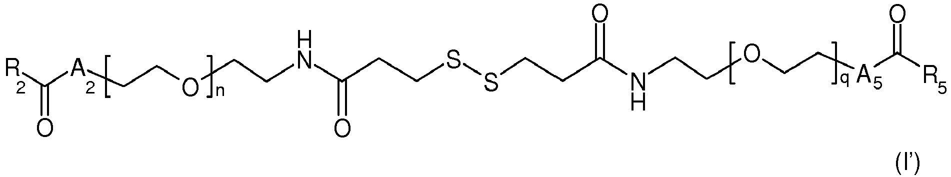 dans laquelle :
- R₂ et R₅ représentent indépendamment une chaîne hydrocarbonée linéaire comprenant de 11 à 23 atomes de carbone, de préférence 17,
- A₂ et A₅ représentent O ou NH, de préférence NH, et
- n et q représentent indépendamment des nombres entiers de 3 à 500, de préférence de 20 à 200.

Dans un mode de réalisation, les groupes H₁ et H₂ sont indépendamment choisis parmi un poly(oxyde d'éthylène) comprenant plus de 3 unités de poly(oxyde d'éthylène), voire plus de 20 unités, notamment plus de 50 (dans les formules susmentionnées, m, n, o, p et/ou q sont de préférence supérieurs à 3, voire 20, notamment plus de 50).

Dans un mode de réalisation, le groupe G du tensioactif de formule (I) de la formulation utilisée comprend une fonction clivable, notamment à certains pH (pH basique ou acide), par des enzymes, par la lumière (lumière visible, ultraviolets ou infrarouge) et/ou au-delà de certaines températures. Généralement, le groupe G comprend alors un groupe G' comprenant une fonction clivable.

Par exemple :
- la fonction ß-cétoaminoester du groupe G de formule (XX) est clivable à pH acide (typiquement autour de 5),
- la fonction disulfure du groupe G de formule (XV) est clivable aux ultraviolets ou par des enzymes telles que des thioréductases,
- la fonction amide du groupe G de formule (XI) est clivable par des enzymes telles que des protéases,
- la fonction phosphate du groupe G de formule (XXII) est clivable par des enzymes telles que des phosphatases,
- la fonction imine des groupes G de formules (XXI) et (XIII) sont clivables à pH acide ou au-delà de certaines températures,
- le cycle cyclohexène du groupe G de formule (XVII) est clivable au-delà de certaines températures (par rétro Diels-Alder),
- la fonction carbonate du groupe G de formule (XIX) et la fonction carbamate du groupe G de formule (XII) sont clivables à pH acide.

L'homme du métier, au vu de ses connaissances générales, sait quelles fonctions sont clivables et dans quelles conditions. Il est notamment à même de choisir la fonction du groupe G' du tensioactif de formule (I) pour qu'elle soit clivable dans les conditions rencontrées lors de l'administration de la formulation selon l'invention.

De préférence, le rapport de la masse de tensioactif de formule (I) sur la masse de l'ensemble (tensioactif de formule (I) / co-tensioactif) est supérieur ou égal à 15%. Il a en effet été observé que de telles formulations sont plus faciles à préparer.

### ● Application thérapeutique

Lorsqu'elle est administrée, la formulation utilisée dans l'invention cible préférentiellement les organes stéroïdiens (ovaires et surrénales).

Sans vouloir être liés à des théories particulières, ce ciblage préférentiel pourrait s'expliquer :
- par le fait que les composés chimiques issus du métabolisme de la formulation utilisée dans l'invention soient utiles comme produits de départ pour synthétiser des hormones, et/ou
- par le fait que la formulation utilisée dans l'invention soit un mimique synthétique de lipoprotéines, qui transportent le cholestérol vers les organes en ayant besoin, tels que les organes stéroïdiens, comme par exemple les VLDL (very low density lipoprotein) ou les chylomycrons. En effet, les gouttelettes de la formulation selon l'invention ont :
   - une taille, et
   - une composition (elles comprennent un dérivé de glycéride (lipide solubilisant) et éventuellement un phospholipide (lipide amphiphile) que l'on retrouve dans les lipoprotéines susmentionnées)
similaires à celles de ces lipoprotéines. Une fois administrées, les gouttelettes de la formulation selon l'invention seraient donc transportées vers les organes stéroïdiens, comme les lipoprotéines.

Comme explicité ci-dessus, la formulation utilisée dans l'invention permet de limiter les doses de l'hormone, de l'agoniste ou de l'antagonsite d'hormone ou du mélange de ceux-ci administrées et permet de limiter les effets secondaires dus à leur administration.

Par ailleurs, en modulant les natures et concentrations des lipides amphiphile et solubilisant, du co-tensioactif, du tensioactif de formule (I) et de l'huile et la taille des gouttelettes de la phase dispersée, il est possible de contrôler la cinétique de délivrance de l'hormone, de l'agoniste ou de l'antagoniste d'hormone, notamment pour éviter les pics de concentrations.

La formulation peut être utilisée telle qu'elle, ou adaptée à l'application visée, par exemple par dilution, pour l'administration de l'hormone, de l'agoniste ou de l'antagoniste d'hormone ou du mélange de ceux-ci, chez l'homme ou chez l'animal.

Du fait qu'elle peut être préparée exclusivement à partir de constituants approuvés pour l'homme, elle est intéressante pour une administration par voie parentérale. Cependant, il est également possible d'envisager une administration par d'autres voies, notamment par voie orale, par voie topique, ou par voie vaginale. Les voies orale et topique (en particulier lorsque la formulation utilisée est sous forme de patch) sont particulièrement préférées.

La formulation utilisée peut être sous forme de gélule, d'ovule, de gel, d'émulsion, de crème, de patch, de spray...

Typiquement, lorsqu'elle est administrée par voie:
- orale, elle est sous forme de gélule ou de capsule,
- buccale et sublinguale, elle est sous forme de gélule, de capsule, de gel, d'émulsion,
- transdermique, elle est sous forme de gel, d'émulsion, de patch, de spray dermique, de spray intranasal,
- intra-utérine, elle est sous forme de gel, de crème, d'ovule,
- intramusculaire, elle est sous forme de gel ou d'émulsion.

Le groupe de patients auquel la formulation est administrée est varié. Bien sûr, pour l'utilisation de la formulation pour la contraception, l'aide à la procréation, le traitement hormonal post-ménopause et le traitement de l'infertilité due à une dysrégulation surrénalienne, les patients sont des femmes. Généralement, pour l'utilisation de la formulation pour le traitement de l'acné, les patients sont des adolescents et/ou des jeunes adultes (typiquement entre 12 et 25 ans).

Une méthode de traitement hormonal comprenant l'administration chez un mammifère, de préférence un humain, qui en a besoin d'une quantité efficace, de la formulation telle que définie ci-dessus est également un des objets de la présente invention.

### ● Préparation de la nanoémulsion utilisée

Avantageusement, les agents sont incorporés dans l'émulsion sous forme de solution, et le solvant est ensuite séparé, par exemple par évaporation. La solution contient l'hormone, l'agoniste ou l'antagoniste d'hormone ou le mélange de ceux-ci en une quantité variable pouvant aller jusqu'à sa limite de solubilité. Le choix du solvant dépend de la solubilité de chaque hormone /agoniste / antagoniste. Les solvants employés peuvent être par exemple le méthanol, l'éthanol, le chloroforme, le dichlorométhane, l'hexane, le cyclohexane, le DMSO, le DMF ou encore le toluène. De préférence, il s'agit de solvants volatils, de préférence non toxiques pour l'homme, de préférence l'éthanol.

La nanoémulsion utilisée peut être préparée aisément par dispersion de quantités appropriées de phase huileuse et de phase aqueuse sous l'effet d'un cisaillement.

Typiquement, on mélange d'abord les différents constituants huileux, l'hormone, l'agoniste ou l'antagoniste d'hormone ou le mélange de ceux-ci pour préparer un pré-mélange huileux pour la phase dispersée de l'émulsion. Le procédé de préparation comprend typiquement les étapes suivantes :
(i) préparer la phase huileuse comprenant un lipide solubilisant, un lipide amphiphile, l'hormone, l'agoniste ou l'antagoniste d'hormone ou le mélange de ceux-ci ;
(ii) préparer une phase aqueuse comprenant un co-tensioactif polyalkoxylé ;
(iii) disperser la phase huileuse dans la phase aqueuse sous l'action d'un cisaillement suffisant pour former une nano-émulsion; et
(iv) récupérer la nano-émulsion ainsi formée.

Le mélange peut éventuellement être facilité par mise en solution d'un des constituants ou du mélange complet dans un solvant organique approprié. Le solvant organique est ensuite évaporé, pour obtenir un pré-mélange huileux homogène pour la phase dispersée.

Par ailleurs, il est préféré de réaliser le pré-mélange (étape (i)) à une température à laquelle l'ensemble des ingrédients est liquide.

Avantageusement, la phase huileuse est dispersée dans la phase aqueuse à l'état liquide. Si l'une des phases se solidifie à température ambiante, il est préférable de réaliser le mélange avec l'une ou de préférence les deux phases chauffées à une température supérieure ou égale à la température de fusion.

L'émulsification sous l'effet de cisaillement est de préférence réalisée à l'aide d'un sonificateur ou d'un microfluidiseur. De préférence, la phase aqueuse puis la phase huileuse sont introduites dans les proportions souhaitées dans un récipient cylindrique approprié puis le sonificateur est plongé dans le milieu et mis en marche pendant une durée suffisante pour obtenir une nanoémulsion, le plus souvent quelques minutes.

On obtient alors une nanoémulsion homogène dans laquelle le diamètre moyen des gouttelettes d'huile est généralement supérieur à 10 nm et inférieur à 200 nm, de préférence entre 30 et 150 nm.

De préférence, le potentiel zêta est inférieur à 20 mV en valeur absolue, c'est-à-dire compris entre -20 mV et 20 mV.

Avant conditionnement, l'émulsion peut être diluée et/ou stérilisée, par exemple par filtration ou dialyse. Cette étape permet d'éliminer les éventuels agrégats qui pourraient s'être formés au cours de la préparation de l'émulsion.

L'émulsion ainsi obtenue est prête à l'emploi, le cas échéant après dilution.

Dans les modes de réalisation dans lesquels la nanoémulsion utilisée comprend un co-tensioactif greffé par une molécule d'intérêt (par exemple un ligand biologique), le procédé de préparation comprend typiquement les étapes suivantes :
(i) préparer la phase huileuse comprenant un lipide solubilisant, un lipide amphiphile, l'hormone, l'agoniste ou l'antagoniste d'hormone ou le mélange de ceux-ci ;
(ii) préparer une phase aqueuse comprenant un co-tensioactif polyalkoxylé et une fonction susceptible de réagir avec la molécule d'intérêt;
(iii) disperser la phase huileuse dans la phase aqueuse sous l'action d'un cisaillement suffisant pour former une nano-émulsion;
(iv) ajouter à la nanoémulsion la molécule d'intérêt dans des conditions permettant le greffage entre la fonction susceptible de réagir avec la molécule d'intérêt du co-tensioactif et la molécule d'intérêt,
(v) récupérer la nano-émulsion ainsi formée.

Le greffage est donc typiquement effectué après la formation de la nanoémulsion, ce qui peut être préconisé lorsque les réactions chimiques employées sont compatibles avec la stabilité colloïdale des émulsions, notamment en termes de pH. De préférence, le pH lors de la réaction de greffage est compris entre 5 et 11.

Dans un autre mode de réalisation, le procédé de préparation comprend les étapes suivantes :
(i) fournir un co-tensioactif polyalkoxylé greffé par la molécule d'intérêt,
(ii) préparer la phase huileuse comprenant un lipide solubilisant, un lipide amphiphile, l'hormone, l'agoniste ou l'antagoniste d'hormone ou le mélange de ceux-ci ;
(iii) préparer une phase aqueuse comprenant le co-tensioactif polyalkoxylé greffé par la molécule d'intérêt;
(iv) disperser la phase huileuse dans la phase aqueuse sous l'action d'un cisaillement suffisant pour former une nano-émulsion;
(v) récupérer la nano-émulsion ainsi formée.

Le co-tensioactif polyalkoxylé greffé par la molécule d'intérêt étant généralement obtenu par la mise en contact de la molécule d'intérêt avec un co-tensioactif comprenant une fonction susceptible de réagir avec la molécule d'intérêt, dans des conditions permettant le greffage entre la fonction susceptible de réagir avec la molécule d'intérêt du co-tensioactif et la molécule d'intérêt.

Dans le mode de réalisation dans lequel la formulation utilisée comprend un tensioactif de formule (I), celle-ci peut être préparée par un procédé comprenant la mise en contact :
- d'une émulsion 1 comprenant une phase aqueuse continue et une phase dispersée sous forme de gouttelettes comprenant un lipide amphiphile et un tensioactif de formule (LI) suivante :

   L₁-X₁-H₁-Y₁-G₁ (LI),
- avec une émulsion 2 comprenant une phase aqueuse continue et une phase dispersée sous forme de gouttelettes comprenant un lipide amphiphile et un tensioactif de formule (LII) suivante :

   G₂-Y₂-H₂₋X₂-L₂ (LII)
où L₁, X₁, H₁, Y₁, L₂, X₂, H₂ et Y₂ sont tels que définis ci-dessus, et G₁ et G₂ sont des groupes susceptibles de réagir pour former le groupe G tel que défini ci-dessus, dans des conditions permettant la réaction des tensioactifs de formules (LI) et (LII) pour former le tensioactif de formule (I) tel que défini ci-dessus, ce par quoi des liaisons covalentes entre les gouttelettes de la phase dispersée se forment.

Lorsque le groupe G comprend un seul groupe G', les groupes G₁ et G₂ sont typiquement des groupes susceptibles de réagir l'un avec l'autre pour former le groupe G.

Lorsque le groupe G comprend plusieurs groupes G', on met généralement les émulsions 1 et 2 en contact avec un composé susceptible de réagir avec les tensioactifs de formules (LI) et (LII) pour former le groupe G. Ce composé comprend typiquement au moins v fonctions G'₁ susceptibles de réagir avec le groupe G₁ et w fonctions G'₂ susceptibles de réagir avec le groupe G₂.

Ainsi, dans le mode de réalisation dans lequel le groupe G répond à la formule -G'-Y₃-G'- définie ci-dessus, le procédé de préparation de la formulation comprend typiquement la mise en contact :
- d'une émulsion 1 telle que définie ci-dessus,
- et d'une émulsion 2 telle que définie ci-dessus,
- avec un composé de formule G'₁-Y₃-G'₂ dans laquelle Y₃ est tel que défini ci-dessus, G'₁ est un groupe susceptible de réagir avec G₁ pour former le premier groupe G' tel que défini ci-dessus et G'₂ est un groupe susceptible de réagir avec G₂ pour former le second groupe G' tel que défini ci-dessus (de nature identique ou différente du premier groupe G'),
dans des conditions permettant la réaction des tensioactifs de formules (LI) et (LII) et du composé de formule G'₁-Y₃-G'₂ pour former le tensioactif de formule (I) dans lequel le groupe G répond à la formule -G'-Y₃-G'- définie ci-dessus, ce par quoi des liaisons covalentes entre les gouttelettes de la phase dispersée se forment.

De même, dans le mode de réalisation défini ci-dessus dans lequel le groupe G est un dendrimère comprenant (v+w) groupes G', le procédé de préparation de la formulation comprend typiquement la mise en contact :
- d'une émulsion 1 telle que définie ci-dessus,
- et d'une émulsion 2 telle que définie ci-dessus,
- avec un dendrimère de formule (G'₁)ᵥ-Y₄-(G'₂)_{w} dans laquelle v et w et sont tels que définis ci-dessus, G'₁ est indépendamment un groupe susceptible de réagir avec G₁ pour former un groupe G' tel que défini ci-dessus et G'₂ est indépendamment un groupe susceptible de réagir avec G₂ pour former un groupe G' tel que défini ci-dessus (chaque G' étant de nature identique ou différente des autres groupes G') et Y₄ est le squelette d'un dendrimère,
dans des conditions permettant la réaction des tensioactifs de formules (LI) et (LII) et du dendrimère de formule (G'₁)ᵥ-Y₄-(G'₂)_{w} pour former le tensioactif de formule (I) dans lequel le groupe G est un dendrimère comprenant (v+w) groupes G', ce par quoi des liaisons covalentes entre les gouttelettes de la phase dispersée se forment.

Par exemple, pour former un groupe G ayant la formule (XXX), (XXXI), (XXXII) et (XXXIII), le composé de formule (G'₁)ᵥ-Y₄-(G'₂)_{w} peut respectivement avoir une des formules (XXX'), (XXXI'), (XXXII') et (XXXIII') suivantes : 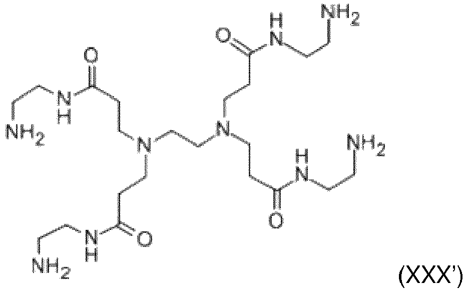 (dans laquelle G'₁ et G'₂ -représentent NH₂ et v et w représentent 2), 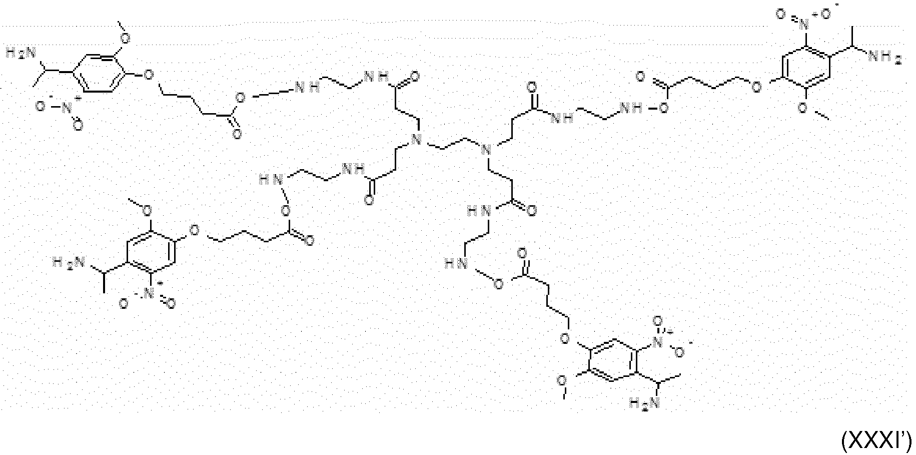 (dans laquelle G'₁ et G'₂ -représentent NH₂ et v et w représentent 2), 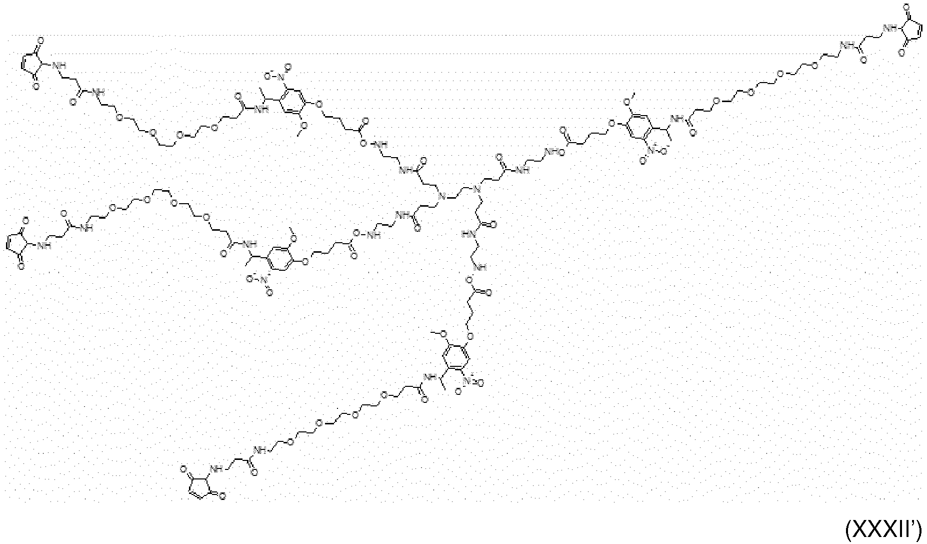 (dans laquelle G'₁ et G'₂ -représentent 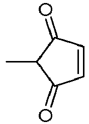 et v et w représentent 2), 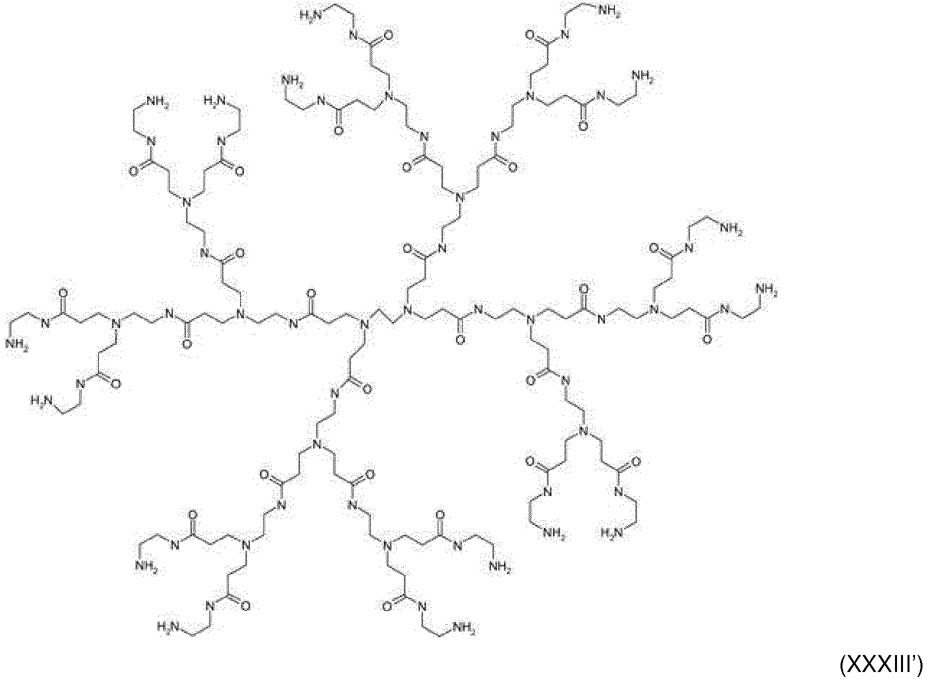 (dans laquelle G'₁ et G'₂ -représentent NH₂ et v et w représentent 8).

Au vu de ses connaissances générales en chimie, l'homme du métier est à même de choisir la nature des groupes G'₁, G'₂, Y₃, Y₄, G₁ et G₂ à utiliser pour former le groupe G et les conditions permettant la réaction. Les réactions de chimie organique usuelles peuvent être suivies, notamment celles décrites dans « Comprehensive Organic Transformations: A Guide to Functional Group Preparations » de Richard C. Larock édité par John Wiley & Sons Inc, et les références qui y sont citées. Ainsi, les exemples de groupes G₁ et G₂ ci-dessous sont cités à titre illustratif et non limitatif.

Typiquement, lorsque le groupe G est constitué d'un groupe G', les groupes G₁ et G₂ des composés de formules (LI) et (LII) peuvent par exemple être choisis comme suit:
- G₁ représente un thiol (-SH) et G₂ représente :
   - soit un maléimide, un tensioactif de formule (I) dans lequel G comprend un groupe G' représentant un groupe de formule (XIV) où A₁₀₂ représente N étant alors formé,
   - soit une vinylsulfone, un tensioactif de formule (I) dans lequel G comprend un groupe G' représentant un groupe de formule (XVI) étant alors formé,
   - soit un groupe -S-S-pyridinyle ou -SH, un tensioactif de formule (I) dans lequel G comprend un groupe G' représentant un groupe de formule (XV) étant alors formé,
- G₁ représente un hydroxyle et G₂ représente -COOH ou un ester activé, un tensioactif de formule (I) dans lequel G comprend un groupe G' représentant un groupe de formule (XXIII) étant alors formé,
- G₁ représente une amine -NH₂ et G₂ représente -COOH ou un ester activé, un tensioactif de formule (I) dans lequel G comprend un groupe G' représentant un groupe de formule (XI) étant alors formé,
- G₁ représente un hydroxyle et G₂ représente un carbonate activé, un tensioactif de formule (I) dans lequel G comprend un groupe G' représentant un groupe de formule (XIX) étant alors formé,
- G₁ représente une amine -NH₂ et G₂ représente un carbonate activé, un tensioactif de formule (I) dans lequel G comprend un groupe G' représentant un groupe de formule (XII) étant alors formé,
- G₁ représente une amine -NH₂ et G₂ représente un aldéhyde -CHO, un tensioactif de formule (I) dans lequel G comprend un groupe G' représentant un groupe de formule (XXI) étant alors formé,
- G₁ représente un hydrazide de formule -(C=O)-NH-NH₂ et G₂ représente un groupe - (C=O)-R10₂, un tensioactif de formule (I) dans lequel G comprend un groupe G' représentant un groupe de formule (XIII) étant alors formé,
- G₁ représente un alcyne et G₂ représente un azide, un tensioactif de formule (I) dans lequel G comprend un groupe G' représentant un groupe de formule (XVIII) étant alors formé,
- G₁ représente un cyclooctyne et G₂ représente un azide, un tensioactif de formule (I) dans lequel G comprend un groupe G' représentant un groupe de formule (XVIII') étant alors formé,
- G₁ représente un furane et G₂ représente un maléimide, un tensioactif de formule (I) dans lequel G comprend un groupe G' représentant un groupe de formule (XVII) étant alors formé,
- G₁ représente un aldéhyde et G₂ représente une amine, un tensioactif de formule (I) dans lequel G comprend un groupe G' représentant un groupe de formule (XXI) étant alors formé,
- G₁ représente un phosphate de formule -O-P(=O)(OH)₂ et G₂ représente un hydroxyle, un tensioactif de formule (I) dans lequel G comprend un groupe G' représentant un groupe de formule (XXII) étant alors formé,
- G₁ représente un bon groupe partant LG et G₂ représente un groupe de formule suivante 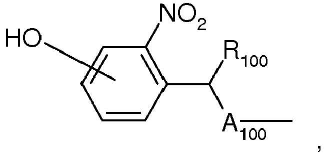 un tensioactif de formule (I) dans lequel G comprend un groupe G' représentant un groupe de formule (XXIV) dans laquelle A₁₀₁ représente O étant alors formé,
- G₁ représente un hydroxyle ou une amine -NH₂ et G₂ représente un groupe de formule suivante 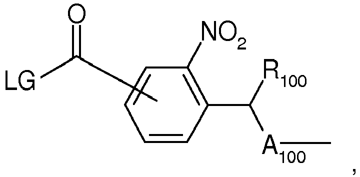 un tensioactif de formule (I) dans lequel G comprend un groupe G' représentant un groupe de formule (XXIV) dans laquelle A₁₀₁ représente respectivement -O-(CO)- ou -NH-(CO) étant alors formé,
- G₁ représente un bon groupe partant LG et G₂ représente un hydroxyle, un tensioactif de formule (I) dans lequel G comprend un groupe G' représentant un groupe de formule (XXV) étant alors formé,
- G₁ représente un bon groupe partant LG et G₂ représente une amine -NH₂, un tensioactif de formule (I) dans lequel G comprend un groupe G' représentant un groupe de formule (XXVI) étant alors formé,
- G₁ représente une oxyamine -O-NH₂ et G₂ représente un aldéhyde, un tensioactif de formule (I) dans lequel G comprend un groupe G' représentant un groupe de formule (XXVII) étant alors formé.

Lorsque le groupe G comprend plusieurs groupes G', le choix des groupes réagissant ensemble : G'₁ et G₁ d'une part et G'₂ et G₂ d'autre part, peut être effectué de la même façon, en remplaçant les groupes G₁ ou G₂ dans les exemples mentionnés ci-dessus par G'₁ ou G'₂.

Les émulsions 1 et 2 mises en oeuvre dans le procédé peuvent être préparées par le premier procédé susmentionné comprenant les étapes (i), (ii), (iii) et (iv).

L'invention sera décrite plus en détail au moyen de la figure en annexe et des exemples qui suivent.

### FIGURE

La figure 1 représente un schéma illustrant le positionnement du tensioactif de formule (I) dans lequel v et w représentent 1 et des gouttelettes de la formulation.
La figure 2 représente un schéma illustrant le positionnement du tensioactif de formule (I) dans lequel v et w représentent 2 et des gouttelettes de la formulation.

### EXEMPLES

### Exemple : Utilisation d'une formulation comprenant de l'oestrogène (oestradiol) ou un agoniste d'oestrogène (éthynil oestradiol)

### ● Préparation et composition des formulations

Six formulations différentes A, B, C, D, E et F ont été réalisées, dont les compositions sont indiquées dans le tableau 2.

**Tableau 1: Composition des formulations**

| | Matière première | Fournisseur | Quantité (pour un lot 2ml) | | | | | | % massique de la formulation totale | |
|---|---|---|---|---|---|---|---|---|---|---|
| | | | A | B | C | D | E | F | | |
| Prémix huileux | Lipoid (mg) | Lipoïd (mg) | 17 | | | | | | 0,85 | 5,4 |
| | Supoccire^{®} NC (mg) | Gattefossé | 68 | 68 | 68 | 68 | 34 | 68 | 3,4 | |
| | Dynasan 114 | Sigma Aldrich | - | - | - | - | 34 | - | | |
| | Huile de soja | Sigma Aldrich | 23 | | | | | | 1,15 | |
| | Oestradiol (mg) | Sigma Aldrich | 1 | 2 | - | - | - | - | 0 ou 0,05 ou 0,1 | |
| | Ethynil Oestradiol (mg) | Sigma Aldrich | - | - | 1 | 2 | 2 | 2 | 0 ou 0,05 ou 0,1 | |
| Phase aqueuse | Myrj® s40 (mg) | Croda | 92 | | | | | | 4,6 | - |
| | PBS 1 X (µL) | Sigma Aldrich | 1800 | | | | | 1800 | 90 | - |

La proportion en phase dispersée des formulations testées (rapport de la masse de [prémix huileux + co-tensioactif PEG] sur la masse de l'ensemble de l'émulsion) est environ de 10%.

Le procédé de préparation suivant a été suivi :
(i) Préparation de la phase huileuse :
   L'huile de soja, la suppocire NC, éventuellement le Dynasan 114, la lécithine, ont été pesés puis mélangées avec du dichlorométhane avant d'être chauffés à 60°C afin d'obtenir une solution visqueuse homogène. Le dichlorométhane permet de favoriser la solubilisation. L'oestradiol ou l'éthynil oestradiol a été dissous dans de l'éthanol puis le volume correspondant à la dose souhaitée a été incorporée dans la solution visqueuse homogène susmentionnée. Les solvants ont ensuite évaporés sous vide.
(ii) Préparation de la phase aqueuse :
   Pendant la phase d'évaporation de l'éthanol, la phase aqueuse a été préparée. Dans un eppendorf de 5ml, le co-tensioactif, du glycérol et la solution aqueuse de PBS ont été mélangés puis dissous dans un bain à 75°C.
(iii) Mélange des deux phases :
   La phase huileuse était à environ 40°C (sous forme visqueuse) et la phase aqueuse à environ 70°C (en sortie du bain). La phase aqueuse a été versée dans la phase huileuse.
(iv) Emulsification :
   Le flacon contenant les deux phases a été fixé dans l'enceinte de sonication. La base du flacon (environ 1cm) a été positionnée dans un bain d'eau à 15°C. La sonotrode conique est plongée dans le flacon, celle-ci étant placée à mi hauteur (sur environ 1 cm dans la solution) pour une meilleure homogénéité de fabrication de la nanoémulsion. La sonication a alors été effectuée avec un sonicateur AV505 (Sonics, Newtown USA). La durée effective de sonication était de 10 min.
(v) Purification :
   La purification a été effectuée par dialyse dans un grand volume de PBS 1X pendant la nuit. Le glycérol est éliminé lors de cette étape. L'échantillon de nanoémulsion purifié a été stérilisé par filtration sur filtre 0,22µm.

### ● Taille et index de polydispersité des gouttelettes des formulations

La taille des gouttelettes des formulations a été mesurée par diffusion quasi-élastique de la lumière ZetaSizer, Malvern dans une solution de PBS 0,1X (Tableau 2).

**Tableau 2 : Taille des gouttelettes et index de polydispersité (PDI) mesurés par diffusion quasi-élastique de la lumière**

| | taille (nm) | PDI |
|---|---|---|
| Formulation A | 52,63 | 0,188 |
| Formulation B | 54,9 | 0,175 |
| Formulation C | 56,09 | 0,188 |
| Formulation D | 52,11 | 0,161 |
| Formulation E | 63,04 | 0,172 |
| Formulation F | 51,13 | 0,115 |

Les expériences ont été reproduites avec des émulsions conservées 30 jours à 4°C après dialyse (tableau 3). Les résultats du tableau 3 montrent que les tailles et PDI sont les mêmes au bout de 30 jours, ce qui montre la stabilité des émulsions dans le temps.

**Tableau 3 : Taille des gouttelettes et index de polydispersité (PDI) mesurés par diffusion quasi-élastique de la lumière des formulations après dialyse ou 30 jours après dialyse**

| | Juste après dialyse | | | | 30j après dialyse | | | |
|---|---|---|---|---|---|---|---|---|
| | taille (nm) | | PDI | | taille (nm) | | PDI | |
| Formulation A | 52,63 | 1,056 | 0,188 | 0,00376 | 53 | 1,063 | 0,2 | 0,011 |
| Formulation B | 54,9 | 1,098 | 0,175 | 0,0035 | 55,94 | 0,9351 | 0,19 | 0,014 |
| Formulation C | 56,09 | 1,1218 | 0,188 | 0,00376 | 57,52 | 0,8764 | 0,19 | 0,016 |
| Formulation D | 42,11 | 0,8422 | 0,161 | 0,00322 | 42,03 | 0,44 | 0,15 | 0,003 |
| Formulation E | 63,04 | 1,2608 | 0,172 | 0,00344 | 64,11 | 1,167 | 0,18 | 0,006 |
| Formulation F | 51,13 | 1,0226 | 0,115 | 0,0023 | 51,04 | 1,161 | 0,15 | 0,0024 |

Les colonnes à droite correspondent à l'écart type pour trois séries de 15 mesures (soit 45 mesures au total).

### ● Potentiel zéta des gouttelettes des formulations

Le potentiel zéta des gouttelettes des formulations a été mesurée par zetasiser, Malvern dans le PBS 0,1X (tableau 4).

**Tableau 4 : Potentiel zéta des formulations**

| | Zeta (mV) |
|---|---|
| Formulation A | -2,71 |
| Formulation B | -4,14 |
| Formulation C | -1,98 |
| Formulation D | -4,89 |
| Formulation E | -6,55 |
| Formulation F | -6,8 |

### ● Dosage HPLC de la quantité d'oestradiol ou d'éthynil oestradiol encapsulé dans les gouttelettes

La quantité d'oestradiol ou d'éthynil oestradiol encapsulé dans chaque formulation est dosée par HPLC (méthode de dosage issue de la Pharmacopée Européenne, avec détection par absorption UV à 230 et 286 nm (HPLC analytique Water Alliance. Température ambiante (25°C), 60% acétonitrile et 40% eau. Colonne phase inverse en C18).

Les taux d'encapsulation et de fuite de l'oestradiol ou de l'éthynil oestradiol ont été évalués par HPLC en dosant l'oestradiol ou ethynil oestradiol avant dialyse (référence 100%), après dialyse (ce qui permet de déduire le taux d'encapsulation) et dans le bain de dialyse (éventuel relargage (« burst release » en anglais)), en utilisant des courbes étalons (dans l'acétonitrile) préalablement réalisées. Les résultats sont regroupés dans le Tableau 5.

**Tableau 5 : Pourcentage d'encapsulation de l'oestradiol ou de l'éthynil oestradiol après dialyse**

| | % d'encapsulation | |
|---|---|---|
| | A(286nm) | A(230nm) |
| Formulation A | 102 | 103 |
| Formulation B | 81 | 86 |
| Formulation C | 96 | 103 |
| Formulation D | 75 | 85 |
| Formulation E | 87 | 86 |
| Formulation F | 100 | 100 |

Ces résultats montrent que l'oestradiol ou l'éthynil oestradiol ne fuient pas hors des gouttelettes après encapsulation (% d'encapsulation entre 85% et 105% suivant le dosage, avec une erreur de +/- 10%, en tenant compte de la méthode d'extraction).

Les expériences ont été reproduites avec des émulsions conservées 30 jours à 4°C après dialyse (tableau 6). La comparaison des données des tableaux 4 et 5 montre qu'aucune fuite de l'oestradiol ou de l'éthynil oestradiol n'a eu lieu, ce qui montre la stabilité des émulsions dans le temps.

**Tableau 6 : Pourcentage d'encapsulation de l'oestradiol ou de l'éthynil oestradiol 30 jours après dialyse**

| | % d'encapsulation | |
|---|---|---|
| | A(286nm) | A(230nm) |
| Formulation A | 116 | 85 |
| Formulation B | 95 | 96 |
| Formulation C | 110 | 117 |
| Formulation D | 108 | 103 |
| Formulation E | 106 | 118 |
| Formulation F | 101 | 107 |

## Revendications

1. Formulation sous forme de nanoémulsion, comprenant une phase aqueuse continue et au moins une phase dispersée, et comprenant :
- un lipide amphiphile,
- un lipide solubilisant comprenant au moins un glycéride d'acides gras,
- une hormone, un agoniste ou un antagoniste d'hormone ou un mélange de ceux-ci, et
- un co-tensioactif comprenant au moins une chaîne composée de motifs d'oxyde d'alkylène,
pour son utilisation pour un traitement hormonal choisi parmi le traitement hormonal post-ménopause, la contraception, l'aide à la procréation, le traitement des ménorragies, le traitement de l'acné, et le traitement des dysrégulations surrénaliennes telles que la maladie d'Addison, la fatigue surrénalienne et l'infertilité.

2. Formulation pour son utilisation selon la revendication 1, dans laquelle :
- l'hormone est choisie parmi :
- l'oestrogène, notamment choisi parmi l'estrone, l'estriol et l'oestradiol,
- la progestérone
- l'hydrocortisone,
- la 5-dehydroepiandrosterone,
- les ganadotrophines,
- la kisspeptine,
- l'agoniste d'hormone est :
- un agoniste d'oestrogène, tel que la tibolone ou un dérivé d'oestrogène choisi parmi les esters d'estrone, d'estriol et d'oestradiol, l'estropipate, le cypionate d'estradiol, les estrogènes conjugués équins et l'éthinyl oestradiol, ou
- un progestatif (agoniste de la progestérone), tel que l'acétate de médroxyprogestérone, le noréthistérone, l'acétate de noréthistérone, le lynesténol, le lévonorgestrel, le norgestrel, le norgestimate, le désogestrel, l'étonogestrel, le gestodène, le nogestimate, le norelgestromine, la nandralone, la 17-hydroxyprogestérone ou la drospirénone,
- l'antagoniste d'hormone est :
- un antiandrogène, tel que l'acétate de cyprotérone, ou
- un antagoniste des oestrogènes, tel que le citrate de clomifène, le tamoxifène, le raloxifène, le toremifène ou le lasofoxifène.

3. Formulation selon la revendication 1 ou 2, pour son utilisation :
- pour la contraception, comprenant :
- de l'oestrogène ou un agoniste d'oestrogène, de préférence de l'éthinyl oestradiol, et/ou
- de la progestérone ou un progestatif, ou
- pour le traitement de l'acné, comprenant :
- un antiandrogène, tel que l'acétate de cyprotérone, et/ou
- un progestatif, tel que l'acétate de médroxyprogestérone, la drospirénone ou le norgestimate, ou
- pour le traitement des ménorragies, comprenant de la progestérone ou un progestatif, ou
- pour le traitement de l'infertilité due à une dysrégulation surrénalienne ou pour l'aide à la fécondation, comprenant du tamoxifène, du citrate de clomifène, des ganadotrophines ou de la kisspeptine, ou
- pour le traitement de la maladie d'Addison comprenant de l'hydrocortisone, ou
- pour le traitement de la fatigue surrénalienne comprenant de l'hydrocortisone ou de la 5-dehydroepiandrosterone, et éventuellement de l'acide pantothénique et/ou de la phosphatidylserine, ou
- pour le traitement hormonal post-ménopause comprenant :
- de l'oestrogène ou un antagoniste d'oestrogène, tel que la tibolone ou un dérivé d'oestrogène choisi parmi les esters d'estrone, d'estriol et d'oestradiol, l'estropipate, le cypionate d'estradiol, les estrogènes conjugués équins et l'éthinyl oestradiol, de préférence de l'éthinyl oestradiol, et
- éventuellement de la progestérone ou un progestatif.

4. Formulation pour son utilisation selon l'une quelconque des revendications 1 à 3, dans laquelle le lipide amphiphile est un phospholipide.

5. Formulation pour son utilisation selon l'une quelconque des revendications 1 à 4, dans laquelle le lipide solubilisant consistant en un mélange de glycérides d'acides gras saturés comportant au moins 10% en poids d'acides gras en C12, au moins 5% en poids d'acides gras en C14, au moins 5% en poids d'acides gras en C16 et au moins 5% en poids d'acides gras en C18.

6. Formulation pour son utilisation selon l'une quelconque des revendications 1 à 5, dans laquelle la phase huileuse comprend au moins une huile.

7. Formulation pour son utilisation selon l'une quelconque des revendications 1 à 6, dans laquelle le co-tensioactif comporte au moins une chaîne composée de motifs d'oxyde d'éthylène ou d'oxyde d'éthylène et d'oxyde de propylène.

8. Formulation pour son utilisation selon l'une quelconque des revendications 1 à 7, comprenant un agent d'imagerie.

9. Formulation pour son utilisation selon l'une quelconque des revendications 1 à 8, dans laquelle la phase dispersée représente entre 35 et 65% en poids par rapport au poids total de la nanoémulsion.

10. Formulation pour son utilisation selon l'une quelconque des revendications 1 à 9, comprenant un tensioactif de formule (I) suivante :
(L₁-X₁-H₁-Y₁)ᵥ-G-(Y₂-H₂-X₂-L₂)_{w} (I),
dans laquelle :
- L₁ et L₂ représentent indépendamment des groupes lipophiles,
- X₁, X₂, Y₁, Y₂ et G représentent indépendamment un groupe de liaison,
- H₁ et H₂ représentent indépendamment des groupes hydrophiles comprenant une chaîne polyalcoxylée,
- v et w sont indépendamment un nombre entier de 1 à 8,
et dans laquelle les gouttelettes de la phase dispersée sont liées entre-elles de façon covalente par le tensioactif de formule (I).
